(19) Europäisches Patentamt
European Patent Office
Office européen des brevets

(11) **EP 3 385 430 A1**

(12) **EUROPEAN PATENT APPLICATION**

(43) Date of publication:
**10.10.2018  Bulletin 2018/41**

(51) Int Cl.:
***D04H 3/013*** (2012.01)     ***B32B 5/26*** (2006.01)

(21) Application number: **17164613.6**

(22) Date of filing: **03.04.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **LENZING AKTIENGESELLSCHAFT**
**4860 Lenzing (AT)**

(72) Inventors:
• **Carlyle, Tom**
  **Spanish Fort, AL Alabama 36527 (US)**

• **Einzmann, Mirko**
  **4600 Wels (AT)**
• **Goldhalm, Gisela**
  **3363 Neufurth (AT)**
• **Hayhurst, Malcolm John**
  **Bulkington, Warwickshire CV12 9RZ (GB)**
• **Mayer, Katharina**
  **4813 Altmünster (AT)**
• **Sagerer Foric, Ibrahim**
  **4840 Vöcklabruck (AT)**

(74) Representative: **Dilg, Haeusler, Schindelmann**
**Patentanwaltsgesellschaft mbH**
**Leonrodstrasse 58**
**80636 München (DE)**

(54) **OPTICALLY TRANSPARENT WET NONWOVEN CELLULOSE FIBER FABRIC**

(57)     A nonwoven cellulose fiber fabric (102), in particular directly manufactured from lyocell spinning solution (104), wherein the fabric (102) comprises a network of substantially endless fibers (108), wherein different ones of the fibers (108) are located at least partially in different distinguishable interconnected layers (200, 202), and wherein the fabric (102) is optically transparent when wet.

Fig. 11

EP 3 385 430 A1

**Description**

Field of the invention

The invention relates to a nonwoven cellulose fiber fabric, a method of manufacturing a nonwoven cellulose fiber fabric, a device for manufacturing a nonwoven cellulose fiber fabric, a product or composite, and a method of use.

Background of the invention

Lyocell technology relates to the direct dissolution of cellulose wood pulp or other cellulose-based feedstock in a polar solvent (for example n-methyl morpholine n-oxide, which may also be denoted as "amine oxide" or "AO") to produce a viscous highly shear-thinning solution which can be transformed into a range of useful cellulose-based materials. Commercially, the technology is used to produce a family of cellulose staple fibers (commercially available from Lenzing AG, Lenzing, Austria under the trademark TENCEL®) which are widely used in the textile industry. Other cellulose products from lyocell technology have also been used.

Cellulose staple fibers have long been used as a component for conversion to nonwoven webs. However, adaption of lyocell technology to produce nonwoven webs directly would access properties and performance not possible for current cellulose web products. This could be considered as the cellulosic version of the meltblow and spunbond technologies widely used in the synthetic fiber industry, although it is not possible to directly adapt synthetic polymer technology to lyocell due to important technical differences.

Much research has been carried out to develop technology to directly form cellulose webs from lyocell solutions (*inter alia,* WO 98/26122, WO 99/47733, WO 98/07911, US 6,197,230, WO 99/64649, WO 05/106085, EP 1 358 369, EP 2 013 390). Further art is disclosed in WO 07/124521 A1 and WO 07/124522 A1.

Object and summary of the invention

It is an object of the invention to provide a cellulose-based fiber fabric having adjustable optical properties and enabling an adjustable functionality.

In order to achieve the object defined above, a nonwoven cellulose fiber fabric, a method of manufacturing a nonwoven cellulose fiber fabric, a device for manufacturing a nonwoven cellulose fiber fabric, a product or composite, and a method of use according to the independent claims are provided.

According to an exemplary embodiment of the invention, a (in particular solution-blown) nonwoven cellulose fiber fabric is provided (which is in particular directly (in particular in an *in situ* process or in a continuous process executable in a continuously operating production line) manufactured from lyocell spinning solution), wherein the fabric comprises a network of substantially endless fibers, wherein different ones of the fibers are located at least partially in different distinguishable interconnected layers, and wherein the fabric is optically transparent when the fabric is wet, i.e. is wetted with a liquid (such as water).

According to another exemplary embodiment, a method of manufacturing (in particular solution-blown) nonwoven cellulose fiber fabric directly from lyocell spinning solution is provided, wherein the method comprises extruding the lyocell spinning solution through one or more jets with orifices supported by a gas flow into a coagulation fluid atmosphere (in particular an atmosphere of dispersed coagulation fluid) to thereby form substantially endless fibers, collecting the fibers on a fiber support unit to thereby form the fabric, and adjusting process parameters so that different ones of the fibers are located at least partially in different distinguishable interconnected layers, and so that the fabric is optically transparent when wet.

According to a further exemplary embodiment, a device for manufacturing (in particular solution-blown) nonwoven cellulose fiber fabric directly from lyocell spinning solution is provided, wherein the device comprises one or more jets with orifices configured for extruding the lyocell spinning solution supported by a gas flow, a coagulation unit configured for providing a coagulation fluid atmosphere for the extruded lyocell spinning solution to thereby form substantially endless fibers, a fiber support unit configured for collecting the fibers to thereby form the fabric, and a control unit (such as a processor configured for executing program code for manufacturing the nonwoven cellulose fiber fabric directly from the lyocell spinning solution) configured for adjusting process parameters so that different ones of the fibers are located at least partially in different distinguishable interconnected layers, and so that the fabric is optically transparent when wet.

According to still another exemplary embodiment, a product or composite is provided which comprises a fabric having the above mentioned properties.

According to yet another embodiment, a nonwoven cellulose fiber fabric having the above-mentioned properties is used for at least one of the group consisting of a wipe, a dryer sheet, a filter, a hygiene product, a medical application product, a geotextile, an agrotextile, clothing, a product for building technology, an automotive product, a furnishing, an industrial product, a product related to beauty, leisure, sports or travel, and a product related to school or office.

In the context of this application, the term "nonwoven cellulose fiber fabric" (which may also be denoted as nonwoven cellulose filament fabric) may particularly denote a fabric or web composed of a plurality of substantially endless fibers. The term "substantially endless fibers" has in particular the meaning of filament fibers having a significantly longer length than conventional staple fibers. In an alternative formulation, the term "substantially endless fibers" may in particular have the meaning of a web formed of filament fibers having a significantly smaller amount of fiber ends per volume than conventional staple fibers. In particular, endless fibers of a fabric having a fabric density of 0.1 t/m$^3$ according to an exemplary embodiment of the invention may have an amount of fiber ends per volume of less than 10,000 ends/cm$^3$, in particular less than 5,000 ends/cm$^3$. For instance, when staple fibers are used as a substitute for cotton, they may have a length of 38 mm (corresponding to a typical natural length of cotton fibers). In contrast to this, substantially endless fibers of the nonwoven cellulose fiber fabric may have a length of at least 200 mm, in particular at least 1000 mm. However, a person skilled in the art will be aware of the fact that even endless cellulose fibers may have interruptions, which may be formed by processes during and/or after fiber formation. As a consequence, a nonwoven cellulose fiber fabric made of substantially endless cellulose fibers has a significantly lower number of fibers per mass compared to nonwoven fabric made from staple fibers of the same denier. A nonwoven cellulose fiber fabric may be manufactured by spinning a plurality of fibers and by attenuating and stretching the latter towards a preferably moving fiber support unit. Thereby, a three-dimensional network or web of cellulose fibers is formed, constituting the nonwoven cellulose fiber fabric. The fabric may be made of cellulose as main or only constituent.

In the context of this application, the term "lyocell spinning solution" may particularly denote a solvent (for example a polar solution of a material such as N-methyl-morpholine, NMMO, "amine oxide" or "AO") in which cellulose (for instance wood pulp or other cellulose-based feedstock) is dissolved. The lyocell spinning solution is a solution rather than a melt. Cellulose filaments may be generated from the lyocell spinning solution by reducing the concentration of the solvent, for instance by contacting said filaments with water. The process of initial generation of cellulose fibers from a lyocell spinning solution can be described as coagulation.

In the context of this application, the term "gas flow" may particularly denote a flow of gas such as air substantially parallel to the moving direction of the cellulose fiber or its preform (i.e. lyocell spinning solution) while and/or after the lyocell spinning solution leaves or has left the spinneret.

In the context of this application, the term "coagulation fluid" may particularly denote a non-solvent fluid (i.e. a gas and/or a liquid, optionally including solid particles) which has the capability of diluting the lyocell spinning solution and exchanging with the solvent to such an extent that the cellulose fibers are formed from the lyocell filaments. For instance, such a coagulation fluid may be water mist.

In the context of this application, the term "process parameters" may particularly denote all physical parameters and/or chemical parameters and/or device parameters of substances and/or device components used for manufacturing nonwoven cellulose fiber fabric which may have an impact on the properties of the fibers and/or the fabric, in particular on fiber diameter and/or fiber diameter distribution. Such process parameters may be adjustable automatically by a control unit and/or manually by a user to thereby tune or adjust the properties of the fibers of the nonwoven cellulose fiber fabric. Physical parameters which may have an impact on the properties of the fibers (in particular on their diameter or diameter distribution) may be temperature, pressure and/or density of the various media involved in the process (such as the lyocell spinning solution, the coagulation fluid, the gas flow, etc.). Chemical parameters may be concentration, amount, pH value of involved media (such as the lyocell spinning solution, the coagulation fluid, etc.). Device parameters may be size of and/or distances between orifices, distance between orifices and fiber support unit, speed of transportation of fiber support unit, the provision of one or more optional *in situ* post processing units, the gas flow, etc.

The term "fibers" may particularly denote elongated pieces of a material comprising cellulose, for instance roughly round or non-regularly formed in cross-section, optionally twisted with other fibers. Fibers may have an aspect ratio which is larger than 10, particularly larger than 100, more particularly larger than 1000. The aspect ratio is the ratio between the length of the fiber and a diameter of the fiber. Fibers may form networks by being interconnected by merging (so that an integral multi-fiber structure is formed) or by friction (so that the fibers remain separate but are weakly mechanically coupled by a friction force exerted when mutually moving the fibers being in physical contact with one another). Fibers may have a substantially cylindrical form which may however be straight, bent, kinked, or curved. Fibers may consist of a single homogenous material (i.e. cellulose). However, the fibers may also comprise one or more additives. Liquid materials such as water or oil may be accumulated between the fibers.

In the context of this document, a "jet with orifices" (which may for instance be denoted as an "arrangement of orifices") may be any structure comprising an arrangement of orifices which are linearly arranged.

In the context of this application, the term "optically transparent" may particularly denote that visible light is at least partially transmitted from one main surface to another opposing another main surface through the fabric. Visible light can be considered as electromagnetic radiation having one or more wavelengths in a range from 400 nm to 800 nm. In other words, the wet fabric is capable to transmit light. However, although a significant portion of the light may pass the wet fabric, there may be also some absorption and/or scattering of light by the fibers.

In the context of this application, the term "wet" may particularly denote that the fabric is filled or soaked with moisture or water or water based solutions or emulsions in the optically transparent state. The mass of moisture or water in the optically transparent fabric may be larger than the mass of the fibers of the fabric.

In the context of this application, the term "different distinguishable interconnected layers" may particularly denote layers of fibers which are visibly and/or mechanically distinguishable on a scanning electromicroscopic image due to the construction within the respective layers and/or at an interface between adjacent layers. The fibers of the different layers may have different fiber properties. Although fibers of one layer and fibers of another layer may be interconnected at an interface plane between the abutting layers, endless fibers of one layer may be substantially free of extending into the respectively other layer. Peeling on the fabric may separate the fabric into the individual layers as a result of a possible weaker connection force at the interface between the layers compared to a stronger adhesion force between fibers within the respective one of the layers.

According to an exemplary embodiment, a nonwoven cellulose fiber fabric is provided which has optically transparent or translucent properties in the visible range when it is wetted with water or moisture. It has turned out that such an optically transparent property of a nonwoven cellulose fiber fabric in a wet condition can be obtained when the process parameters during a manufacturing process are adjusted accordingly. Firstly, the fact that the fabric is composed of endless fibers promotes the optical transparency, because it keeps the number of fiber ends (acting as scattering centers) within the fabric very small. Secondly, further measures can be taken in terms of the manufacturing process control to obtain a fiber network which has pronounced optically transparent properties. For instance fiber diameters, fiber cross-sectional shape, a merging characteristic between fibers of the fiber network, etc., can be adjusted correspondingly. According to the process of embodiments of the invention high-purity nonwoven fabrics (in particular in terms of heavy metals content) can be achieved. Even more advantageous, it has turned out to be possible to manufacture a multilayer fabric which has a higher thickness than a single layer fabric and which also has an interconnection interface between the layers, which multilayer property nevertheless does not deteriorate the transparency of the fabric as a whole. Adjusting process parameters during manufacture of such a multilayer fabric allows to obtain such an optical transparency in the wet state of the fiber network, since in particular formation of the interface between the layers by cellulose-based merging positions (rather than by a separate binder material) promotes undisturbed propagation of visible electromagnetic radiation through the multilayer fabric. As a result, the interface between the layers does not involve a significant amount of additional scattering centers which would deteriorate optical transparency significantly. Highly advantageously, such a multilayer fabric may be functionalized separately for the different layers so that a fabric with a high degree of functionality may be obtained.

Detailed description of embodiments of the invention

In the following, further exemplary embodiments of the nonwoven cellulose fiber fabric, the method of manufacturing a nonwoven cellulose fiber fabric, the device for manufacturing a nonwoven cellulose fiber fabric, the product or composite, and the method of use are described.

In an embodiment, the multiple layers of the fabric may be formed with a plurality of jets which may for instance be arranged serially along a transport direction of the fabric. Each of the jets may form a respective one of the layers. However, alternatively, it is for instance possible that the fabric is first formed with the first layer only. Subsequently, this layer may be transported again along the fiber transport unit, and a second layer may be formed on the first layer with the same jet. This procedure can be repeated as many times as layers are required. In the latter embodiment, a single jet may be sufficient in the device.

In an embodiment, the fabric is optically opaque or non-transparent when the fabric is dry, i.e. the fibers and the gaps between the fibers do not contain moisture. Thus, visible light having one or more wavelengths in a range from 400 nm to 800 nm cannot be transmitted from one main surface to an opposing another main surface through the fabric as efficiently as in the wet state when the fabric is dried out. In other words, the dry fabric is not capable to substantially transmit light with high efficiency, but will scatter and/or absorb a significant amount of the visible light impinging on the dry fabric.

In an embodiment, the fabric has an optical gray value of at least 90, in particular of at least 100 (in particular on a scale from 0 to 255), when wet. Correspondingly, the fabric may be optically opaque, in particular with an optical gray value of lower than 85 (in particular on a scale from 0 to 255), when completely dry. More specifically, the fabric may have an optical transmittance gray value at maximum number of pixel of more than 90, in particular of more than 100 (on a scale from 0 to 255), when wet. Accordingly, the fabric may have an optical transmittance gray value at maximum number of pixel of less than 85 (on a scale from 0 to 255), in particular of less than 80 (on a scale from 0 to 255), when dry. The mentioned high values of the optical gray value of at least 90, in particular of at least 100, which can be obtained with a nonwoven cellulose fiber fabric according to an exemplary embodiment of the invention make sure that a high portion of the impinging light in fact transmits the wet fabric.

The gray value or gray level indicates the brightness of a pixel on an image of the fabric detected in a transmission measurement. Thus, an electromagnetic radiation source may emit electromagnetic radiation in the optically visible range which is directed towards a first main surface of the fabric. A light detector capable of detecting visible light is arranged on an opposing second main surface of the fabric and measures the visible electromagnetic radiation which has been transmitted through the fabric (in the respective humidity state of the fabric, i.e. wet or dry). The detector may have multiple pixels, which may for instance be arranged in a matrix-like pattern (for instance may be a CCD detector or a CMOS detector).

The minimum gray level is 0. The maximum gray level depends on the digitization depth of the image. For an 8-bit-deep image, the maximum gray level is 255. In a grayscale or color image a pixel can take on any value between 0 and 255.

In a color image, the gray value or gray level of each pixel can be calculated using the following formula:

$$\text{Gray value} = 0.299 * \text{red component} + 0.587 * \text{green component} + 0.114 * \text{blue component}$$

This formula takes into account the color sensitivity of the human eye making the presentation of the gray levels independent of color and limited solely to the brightness of the individual pixels.

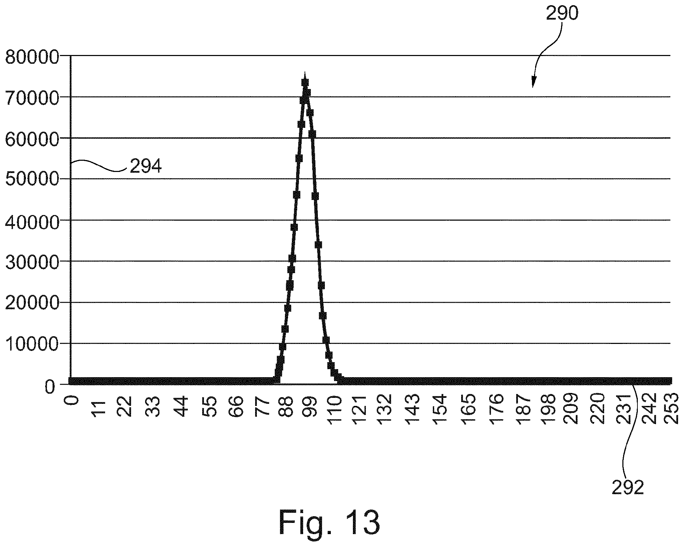

A gray level histogram (compare Figure 13) indicates how many pixels of an image share the same gray level. The x-axis shows the gray levels (in particular from 0 to 255), the y-axis shows their number (which is indicative of their frequency) in the image. This information can be used to calculate a threshold value or a maximum value.

The gray values mentioned in the present document for the dry fabric and the opaque fabric, respectively, according to an exemplary embodiment of the invention relate to the maximum of the curve in such a gray level histogram.

In an embodiment, the fabric is optically transparent when being wet to such a degree that a mass ratio between a mass of moisture in an interior of the fabric and a mass of the fibers (i.e. the cellulose only) is at least 3, in particular is at least 5, more particularly is at least 7. In a preferred embodiment, the maximum water holding capability is above 700 %.

The mass percentage of the water absorbing capability indicates the ratio between the absorbable water mass and the dry fiber mass. It is mentioned that the described water absorbing capability is often also denominated water holding value.

The described water holding capability values relate to measurements which are carried out in accordance with the standard DIN 53 923 (F36_3) and relate to fabrics having a mass per unit area respectively a grammage in the range between 16 g/m$^2$ and 38 g/m$^2$ (gram per square meter). Specifically, the water holding capability values relate to measurements which are carried at in the presence of standard climate conditions. Fully dry hereby means that after manufacturing the fabric (which includes a drying) the fabric has been conditioned for 24 hours at a standard climate being defined with a temperature of 23°C $\pm$ 2°C, with a relative humidity of 50% $\pm$ 5%. All measurement, unless notified otherwise, have been performed under this standard climate.

In an embodiment, fibers of a respective layer are integrally merged at at least one merging position within said layer. In particular, at least part of (in particular at least 10% of) the fibers are integrally merged at merging positions. In the context of this application, the term "merging" may particularly denote an integral interconnection of different fibers at the respective merging position which results in the formation of one integrally connected fiber structure composed of the previously separate fiber preforms. Merging may be denoted as a fiber-fiber connection being established during coagulation of one, some or all of the merged fibers. Interconnected fibers may strongly adhere to one another at a respective merging position without a different additional material (such as a separate adhesive) so as to form a common structure. Separation of merged fibers may require destruction of the fiber network or part thereof. According to the described embodiment, a nonwoven cellulose fiber fabric is provided in which some or all of the fibers are integrally connected to one another by merging. Merging may be triggered by a corresponding control of the process parameters of a method of manufacturing the nonwoven cellulose fiber fabric. In particular, coagulation of filaments of lyocell spinning solution may be triggered (or at least completed) after the first contact between these filaments being not yet in the precipitated solid fiber state. Thereby, interaction between these filaments while still being in the solution phase and then or thereafter converting them into the solid-state phase by coagulation allows to properly adjust the merging characteristics. A degree of merging is a powerful parameter which can be used for adjusting the properties of the manufactured fabric. In a preferred embodiment, merging between fibers is triggered by bringing different fiber preforms in form of lyocell spinning solution in direct contact with one another prior to coagulation.

In an embodiment, fibers of different layers are integrally merged at at least one merging position between said layers. Hence, different ones of the fibers being located at least partially in different distinguishable layers (which may be identical or which may differ concerning one or more parameters such as merging factor, fiber thickness, etc.) may be integrally connected at at least one merging position. For instance, two (or more) different layers of a fabric may be formed by serially aligning two (or more) jets with orifices through which lyocell spinning solution is extruded for coagulation and fiber formation. When such an arrangement is combined with a moving fiber support unit (such as a conveyor belt with a fiber accommodation surface), a first layer of fibers is formed on the fiber support unit by the first jet, and the second jet forms a second layer of fibers on the first layer when the moving fiber support unit reaches the position of the second jet. The process parameters of this method may be adjusted so that merging points are formed between the first layer and the second layer. In particular, fibers of the second layer under formation being not yet fully cured or solidified by coagulation may for example still have exterior skin or surface regions which are still in the liquid lyocell solution phase and not yet in the fully cured solid state. When such pre-fiber structures come into contact with one another and fully cure into the solid fiber state thereafter, this may result in the formation of two merged fibers at an interface between different layers. The higher the number of merging positions, the higher is the stability of the interconnection between the layers of the fabric. Thus, controlling merging allows to control rigidity of the connection between the layers of the fabric. Merging can be controlled, for example, by adjusting the degree of curing or coagulation before pre-fiber structures of a respective layer reach the fiber support plate on an underlying layer of fibers or pre-fiber structures. By merging of fibers of different layers at an interface there between, undesired separation of the layers may be prevented. In the absence of merging points between the layers, peeling off one layer from the other layer of fibers may be made possible.

In an embodiment, the merging between the different layers is adjusted so that pulling on the layers in opposite directions results in a separation of the fabric at an interface between the different layers. This can be achieved when the merging is adjusted so that a merging-based connection force between the different layers is smaller than a merging based connection force within a respective one of the different layers. In particular, a number of merging points or merging positions per volume may be larger in an interior of a respective one of the connected layers than at in an interface region between the layers. A corresponding fabric can be manufactured by controlling the relation between inter-layer coagulation and *intra*-layer coagulation.

Highly advantageously, *inter*-layer merging and/or *intra*-layer merging may be accomplished by integral cellulose connections rather than by adding an additional binder or adhesive material. This has the advantage that no additional scattering centers are added to the fabric which would deteriorate optically transparency in the wet state of the fabric.

In an embodiment, the fabric comprises a permanently opaque (i.e. being always opaque regardless of the humidity state of the fabric) marker being optically visible through the fabric when a moisture content of the fabric is above a predetermined threshold value and being optically invisible through the fabric when the moisture content of the fabric falls below the predetermined threshold value, in particular when the fabric is or becomes dry. Such an opaque marker may be optically non-transparent in all humidity states of the fabric. Such an opaque marker, which may also be denoted as opaque indication, may for instance be printed with ink on the fiber network. The opaque marker may be located at an exterior surface of the fabric, at an interface between different layers of the fabric, or in an interior of one of the layers. The opaque marker can for instance be an alphanumerical code. When the fabric is wet, the marker will be visible for a user. When however the fabric has dried out, the fabric turns from optically transparent into opaque and the marker is no longer visible for the user. In the example of a face mask for instance, completion of the release of an aqueous active agent previously accommodated within the multilayer fabric can be indicated to a user by the vanishing marker.

In an embodiment, the fabric comprises three interconnected layers composed of two opposing cover layers between which an intermediate layer is embedded, wherein an active agent is mainly accommodated in the intermediate layer and is releasable via at least one of the cover layers towards an environment. In the context of this application, the term "active agent" may particularly denote a substance that may produce a chemical reaction or may have a physical impact, that may have, in turn, an impact on the physical (for instance mechanical, electrical, magnetic, optical, etc.) properties of the fabric or an environment thereof, and/or that may have a biological impact (for instance a medical impact, so that the active agent may for instance be a pharmaceutically active agent). The active agent may comprise or consist of one or more solid particles and/or one or more liquids. Such an embodiment may be advantageous for pharmaceutical, medical and cosmetic applications. The active agent may be accommodated protected in the intermediate layer, and can be supplied to a desired destination (for instance the skin of a user wearing a face mask).

In an embodiment, an adhesion force between the distinguishable interconnected layers is smaller than an adhesion force within a respective one of the layers. This allows to separate the multilayer fabric in a predictive way along the layer interface.

In an embodiment, an average diameter of the fibers of a respective layer is different from an average diameter of the fibers of a respective other layer. For instance, a ratio between the average diameter of the fibers of the one layer and the average diameter of the fibers of the other layer may be at least 1.5, in particular may be at least 2.5, more particularly may be at least 4. Thus, a nonwoven cellulose fiber fabric may be provided which can be manufactured as a network of substantially endless cellulose fibers showing a pronounced inhomogeneity in terms of fiber diameter between different layers (but additionally or alternatively also within one layer). It has turned out that the distribution of diameters of the fibers of the nonwoven cellulose fiber fabric is a powerful design parameter for adjusting the physical properties of the obtained fabric. However, it should be mentioned that by varying fiber diameter as a design parameter for a fabric, fiber physics may be adjusted in a more general way allowing to vary physical properties of the fabric over a broad range (wherein reinforcing stiffness is only one option or example). For instance, fiber diameter variation can also be a powerful tool for tuning moisture management of the manufactured fabric.

In an embodiment, an interconnection between the layers is accomplished without separate binder or glue material. Additionally or alternatively, an interconnection between fibers within a respective one of the layers may be accomplished without separate binder or glue material. In contrast to this, the interconnections may be made by cellulose material itself. Thus, the merging positions may be formed by cellulose material resulting directly from the coagulation of lyocell spinning solution. This not only renders the separate provision of a fiber connection material (such as an adhesive or a binder) dispensable, but also keeps the fabric clean and made substantially of a single material. Moreover, a separate binder or glue material may introduce additional scatter centers into the fabric. As a result of the omission of such an additional non-cellulose material, optical transparency of the fabric in the wet state can be further enhanced.

In an embodiment, the endless fibers of a nonwoven fabric of an embodiment of the invention with a density of $0.1$ t/m$^3$ have an amount of fiber ends per volume of less than $10,000$ ends/cm$^3$, in particular less than $5,000$ ends/cm$^3$. Since the fibers of the nonwoven cellulose fiber fabric according to an exemplary embodiment of the invention are endless fibers, the number of (in practice not completely unavoidable, as known by a skilled person) fiber ends of the fabric may be very small. In contrast to this, conventional staple fibers may have significantly higher numbers of fiber ends per volume. It has turned out that free fiber ends may serve as scattering centers limiting the optical transparency of the fabric in the wet state. Therefore, the use of endless fibers with very small amount of free ends is particularly appropriate for obtaining a highly optically transparent property of the wet fabric.

In an embodiment, at least 50%, in particular at least 80%, of the fibers have a cross sectional shape having a roundness of more than 60%, in particular of more than 80%. In the context of this application, the term "roundness" may particularly denote the ratio between the inscribed circle and the circumscribed circle of a cross section of a fiber, i.e. the maximum and minimum sizes for circles that are just sufficient to fit inside and to enclose the shape of the fiber's cross section. For determining roundness, a cross sectional plane perpendicular to an extension direction of the fiber may be intersected with the fiber. Thus, roundness may be denoted as the measure of how closely the cross sectional shape of a respective fiber approaches that of a circle having a roundness of 100%. For example, a cross-section of the respective fiber may have an oval (in particular elliptic) shape or may have a polygonal shape was however only small deviations from a circular cross-section. It has turned out that the optical transmissivity of a fiber network with perfectly circular cylindrical fibers (i.e. fibers having a circular cross-section) is higher than the optical transmissivity of fibers having a flat or irregular cross-section. It is believed that such irregularities or deviations from a circular cross-section may involve additional scattering centers in the fabric which may deteriorate optical transparency or translucency. In order to suppress a corresponding deterioration of the optical transmission of light by the fiber fabric, it is a powerful tool so adjust the process parameters of the manufacturing method so that the deviation of the cross-section of the fibers from a circular geometry is as small as possible. For instance, the orifices or openings of nozzles through which a lyocell spinning solution is extruded prior to precipitation may be constructed circular.

In an embodiment, the fabric is configured so that a wicking speed in the first 10 seconds is at least 0.25 g water/g fabric/s. More particularly, the wicking speed in the first 10 seconds may be at least 0.35 g water/g fabric /s, in particular at least 0.4 g water/g fabric /s. The wicking speed may correspond to the velocity according to which a medium is soaked from an exterior of the fabric into an interior thereof. By correspondingly adjusting the process parameters of the method of manufacturing nonwoven cellulose fiber fabric according to an exemplary embodiment of the invention, it is possible to obtain a fabric soaking medium (in particular moisture) very quickly. Also a liquid spreading speed may be correspondingly high. This may be highly advantageous for certain applications such as wipes, hygiene products or active agent release fabric. In terms of the optical properties of the fabric, a high wicking speed may correspond to the possibility of rapidly switching the fabric from an optically opaque into an optically transparent state, and *vice versa.* Thereby, an optical switch may be formed, wherein the switching operation corresponds to the supply of moisture to or the removal of moisture from the fabric.

The values for the wicking speed described in this document relate to a "wicking speed test" wherein the sample under test (i.e. the respective fabric) is conditioned in a fully dry state. Fully dry means that after manufacturing the fabric (which includes a drying) the fabric has been conditioned for 24 hours at a standard climate being defined with a temperature of $23°C \pm 2°C$, with a relative humidity of $50\% \pm 5\%$. All measurement, unless notified otherwise, have been performed under this standard climate. In the "wicking speed test" the sample under test is placed onto a test table. At its center, the test table is connected via an opening and a channel with a liquid reservoir. The liquid reservoir is filled with fully distilled water. The height of the test table corresponds exactly to the filling level of the water within the liquid reservoir. Thereby, it is ensured that no hydrostatic pressure is present and the suction respectively the wicking of the sample under test is generated exclusively by the suction power of the sample under test. During the actual "wicking speed test" the volume of the water, which is absorbed by the sample under test, is continuously refilled into the liquid reservoir with a syringe. This means that the level of the liquid is always kept constant. The volume of the refilled water is converted into the mass of the refilled water (via the known density of mass of distilled water). It is obvious that with this procedure the wicking speed decreases with time because the suction power of the sample under test decreases with an increasing "absorption load" of the sample under test caused by the absorbed water. The procedure of refilling is continued until for the refilling of water a threshold value of 0.005g per 20 seconds is reached. A measurement curve depicting the mass of added water as a function of time is recorded and evaluated. In this document the wicking speed in g water/g fabric/s is the slope of this measurement curve with a first time slot of 10 seconds starting from the beginning of the actual test.

In an embodiment, at least 80 or even at least 97 mass percent of the fibers have an average fiber diameter in a range between 1 $\mu$m and 40 $\mu$m, in particular between 3 $\mu$m and 15 $\mu$m. By the described method and when adjusting the process parameters accordingly, also fibers with very small dimensions (also in a range between 3 $\mu$m and 5 $\mu$m, or below) may be formed. With such small fibers, a fabric with a smooth surface may be formed which is nevertheless rigid as a whole. The mentioned relatively small fiber diameters have turned out to further promote optical transparency in the wet state.

In an embodiment, the fabric is configured as a lotion delivery system. In view of the biocompatible properties of the manufactured fabric due to the low heavy metal contamination (see for instance the anti-allergic effect of a low nickel contamination), the manufactured fabric is highly appropriate for cosmetic applications such as a lotion delivery system. Lotion may be stored or retained in an interior of the fabric and may be released in a predictable and reproducible way. Delivering lotion corresponds to removing moisture from an interior of the fabric, so that completion of the lotion delivery (for instance to a human skin) may be easily recognized visually by a user at a result of a change of the fabric from the wet transparent state into the dry opaque state.

In an embodiment, the fiber structure of the nonwoven fabric is controlled in a way to tailor different functions, in particular at least one of the group consisting of wicking, anisotropic behavior, oil retention, water retention, cleanability, roughness, and mechanical properties. Such a functionalization may be obtained by an adjustment of physical properties of the fibers and the fabric composed thereof, in particular by an adjustment of a merging factor, an adjustment of a multilayer configuration of a fabric, an adjustment of fiber thickness and an adjustment of the fiber density corresponding to number and dimension of hollow spaces in an interior of the fabric. Secondly, such a functionalization be performed with a multilayer fabric so that, in an embodiment, the fibers located in multiple layers may be provided with different functionalities. Different functionalities of different layers may be the result of different fiber diameters and/or different fiber diameter distributions and/or different fiber density and/or merging properties. For example, the (in particular different) functionalities may be wicking properties (in particular different fluid distribution properties when sucking fluid), anisotropic behavior (in particular different mechanical, chemical and/or hydrodynamic properties in different directions of the fabric), oil absorbing capability (in particular a strong capability of absorbing oil in one layer, and a lower oil absorbing capability in another layer), water absorbing capability (in particular a strong capability of absorbing water in one layer, and a lower water absorbing capability in another layer), cleanability (in particular a stronger capability of cleaning dirt from a surface by the fabric in one layer, and a less pronounced capability of cleaning in another layer), and/or roughness (for instance one rougher surface layer and one smoother surface layer).

In an embodiment, the fibers have (in particular the fiber fabric has) a copper content of less than 5 ppm (in particular 5 mass ppm, i.e. 5 mg/kg) and/or have a nickel content of less than 2 ppm (in particular 2 mass ppm, i.e. 2 mg/kg). Due to the use of a lyocell spinning solution as a basis for the formation of the endless fiber-based fabric (in particular when involving a solvent such as N-methyl-morpholine, NMMO), the contamination of the fabric with the mentioned particularly harmful heavy metals copper (which may be harmful to health for human beings, in particular for children, when exceeding a certain dose) and/or nickel (which may cause allergic reactions of a user) may be kept extremely small. In particular, the very small amount of copper contamination can be ensured by omitting a copper salt solution for preparing the spinning solution. The low contamination of the fabric with heavy metals also keeps the amount of non-cellulose scattering centers in the fabric small, thereby contributing to the high optical transparency and the wet condition. Thus, adapting the process parameters of the manufacturing method so that the contamination with heavy metals is small further improves the light transmission capability of the fabric when wet. This can be accomplished for instance by providing the operating fluids of the fiber manufacturing method (such as lyocell spinning solution, coagulation fluid, wash liquor, gas flow) free of heavy metal compound (such as copper salt solution, as used in conventional approaches). Additionally or alternatively, this can be accomplished by preventing the lyocell spinning solution or the manufactured fabric free of a contact with heavy metals sources.

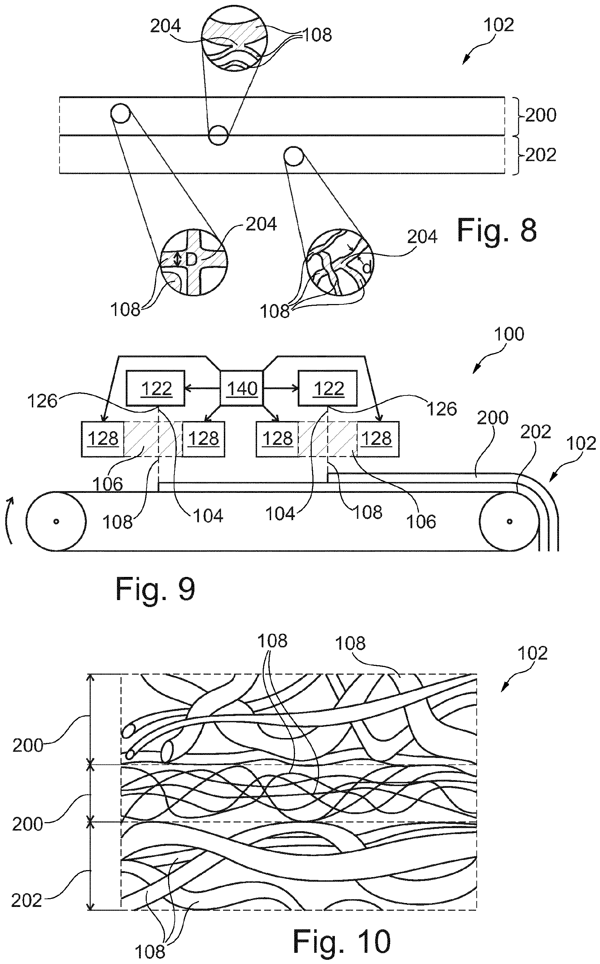

In an embodiment, the device comprises at least one further jet with orifices configured for extruding further lyocell spinning solution supported by a further gas flow, the further jet being arranged downstream (in a fiber transport direction of the fiber support unit) of the jet, and wherein the array is configured for forming one of the layers and the further jet is configured for forming another one of the layers on the layer. A corresponding embodiment is shown in Figure 9. The described embodiment has the advantage that the properties of the different layers of the multilayer fabric may be adjusted completely separately and independently.

In an embodiment, the method further comprises further processing the fibers and/or the fabric after collection on the fiber support unit but preferably still *in situ* with the formation of the nonwoven cellulose fiber fabric with endless fibers. Such *in situ* processes may be those processes being carried out before the manufactured (in particular substantially endless) fabric is stored (for instance rolled by a winder) for shipping to a product manufacture destination. For instance, such a further processing or post processing may involve hydroentanglement. Hydroentanglement may be denoted as a bonding process for wet or dry fibrous webs, the resulting bonded fabric being a nonwoven. Hydroentanglement may use fine, high pressure jets of water which penetrate the web, hit a fiber support unit (in particular a conveyor belt) and bounce back causing the fibers to entangle. A corresponding compression of the fabric may render the fabric more compact and mechanically more stable. Additionally or alternatively to hydroentanglement, steam treatment of the fibers with a pressurized steam may be carried out. Additionally or alternatively, such a further processing or post processing may involve a needling treatment of the manufactured fabric. A needle punching system may be used to bond the fibers of the fabric or web. Needle punched fabrics may be produced when barbed needles are pushed through the fibrous web forcing some fibers through the web, where they remain when the needles are withdrawn. If sufficient fibers are suitably displaced the web may be converted into a fabric by the consolidating effect of these fibers plugs. Yet another further processing or post processing treatment of the web or fabric is an impregnating treatment. Impregnating the network of endless fibers may involve the application of one or more chemicals (such as a softener, a hydrophobic agent, an antistatic agent, etc.) on the fabric. Still another further processing treatment of the fabric is calendering. Calendering may be denoted as a finishing process for treating the fabric and may employ a calender to smooth, coat, and/or compress the fabric.

A nonwoven cellulose fiber fabric according to an exemplary embodiment of the invention may also be combined (for instance *in situ* or in a subsequent process) with one or more other materials, to thereby form a composite according to an exemplary embodiment of the invention. Exemplary materials, which can be combined with the fabric for forming such a composite may be selected from a group of materials comprising, but not being limited to, the following materials or combinations thereof: fluff pulp, a fiber suspension, a wetlaid nonwoven, an airlaid nonwoven, a spunbond web, a meltblown web, a carded spunlaced or needlepunched web or other sheet like structures made of various materials. In an embodiment, the connection between the different materials can be done by (but not limited to) one or a combination of the following processes: merging, hydroentanglement, needle punching, hydrogen bonding, thermobonding, gluing by a binder, laminating, and/or calendering.

In the following, exemplary advantageous products comprising, or uses of, a nonwoven cellulose fiber fabric according to exemplary embodiments of the invention are summarized:

Particular uses of the webs, either 100% cellulose fiber webs, or for example webs comprising or consisting of two or more fibers, or chemically modified fibers or fibers with incorporated materials such as anti-bacterial materials, ion exchange materials, active carbon, nano particles, lotions, medical agents or fire retardants, or bicomponent fibers may be as follows:

The nonwoven cellulose fiber fabric according to exemplary embodiments of the invention may be used for manufacturing wipes such as baby, kitchen, wet wipes, cosmetic, hygiene, medical, cleaning, polishing (car, furniture), dust, industrial, duster and mops wipes.

It is also possible that the nonwoven cellulose fiber fabric according to exemplary embodiments of the invention is used for manufacturing a filter. For instance, such a filter may be an air filter, a HVAC, air condition filter, flue gas filter, liquid filters, coffee filters, tea bags, coffee bags, food filters, water purification filter, blood filter, cigarette filter; cabin filters, oil filters, cartridge filter, vacuum filter, vacuum cleaner bag, dust filter, hydraulic filter, kitchen filter, fan filter, moisture exchange filters, pollen filter, HEVAC/HEPA/ULPA filters, beer filter, milk filter, liquid coolant filter and fruit juices filters.

In yet another embodiment, the nonwoven cellulose fiber fabric may be used for manufacturing absorbent hygiene products. Examples thereof are an acquisition layer, a coverstock, a distribution layer, an absorbent cover, sanitary pads, topsheets, backsheets, leg cuffs, flushable products, pads, nursing pads, disposal underwear, training pants, face masks, beauty facial masks, cosmetic removal pads, washcloths, diapers, and sheets for a laundry dryer releasing an active component (such as a textile softener).

In still another embodiment, the nonwoven cellulose fiber fabric may be used for manufacturing a medical application product. For instance, such medical application products may be disposable caps, gowns, masks and shoe cover, wound care products, sterile packaging products, coverstock products, dressing materials, one way clothing, dialyses products, nasal strips, adhesives for dental plates, disposal underwear, drapes, wraps and packs, sponges, dressings and wipes, bed linen, transdermal drug delivery, shrouds, underpads, procedure packs, heat packs, ostomy bag liners, fixation tapes and incubator mattresses.

In yet another embodiment, the nonwoven cellulose fiber fabric may be used for manufacturing geotextiles. This may involve the production of crop protection covers, capillary matting, water purification, irrigation control, asphalt overlay, soil stabilisation, drainage, sedimentation and erosion control, pond liners, impregnation based, drainage channel liners, ground stabilisation, pit linings, seed blankets, weed control fabrics, greenhouse shading, root bags and biodegradable plant pots. It is also possible to use the nonwoven cellulose fiber fabric for a plant foil (for instance providing a light protection and/or a mechanical protection for a plant, and/or providing the plant or soil with dung or seed).

In another embodiment, the nonwoven cellulose fiber fabric may be used for manufacturing clothing. For example, interlinings, clothing insulation and protection, handbag components, shoe components, belt liners, industrial headwear/foodwear, disposable workwear, clothing and shoe bags and thermal insulation may be manufactured on the basis of such fabric.

In still another embodiment, the nonwoven cellulose fiber fabric may be used for manufacturing products used for building technology. For instance, roofing and tile underlay, underslating, thermal and noise insulation, house wrap, facings for plaster board, pipe wrap, concrete moulding layers, foundations and ground stabilisation, vertical drainages, shingles, roofing felts, noise abatement, reinforcement, sealing material, and damping material (mechanical) may be manufactured using such fabric.

In still another embodiment, the nonwoven cellulose fiber fabric may be used for manufacturing an automotive product. Examples are a cabin filter, boot liners, parcel shelves, heat shields, shelf trim, moulded bonnet liners, boot floor covering, oil filter, headliners, rear parcel shelves, decorative fabrics, airbags, silencer pads, insulation materials, car covers, underpadding, car mats, tapes, backing and tufted carpets, seat covers, door trim, needled carpet, and auto carpet backing.

Still another field of application of fabric manufactured according to exemplary embodiments of the invention are furnishings, such as furniture, construction, insulator to arms and backs, cushion thicking, dust covers, linings, stitch reinforcements, edge trim materials, bedding constructions, quilt backing, spring wrap, mattress pad components, mattress covers, window curtains, wall coverings, carpet backings, lampshades, mattress components, spring insulators, sealings, pillow ticking, and mattress ticking.

In yet another embodiment, the nonwoven cellulose fiber fabric may be used for manufacturing industrial products. This may involve electronics, floppy disc liners, cable insulation, abrasives, insulation tapes, conveyor belts, noise absorbent layers, air conditioning, battery separators, acid systems, anti-slip matting stain removers, food wraps, adhesive tape, sausage casing, cheese casing, artificial leather, oil recovery booms and socks, and papermaking felts.

Nonwoven cellulose fiber fabric according to exemplary embodiments of the invention is also appropriate for manufacturing products related to leisure and travel. Examples for such an application are sleeping bags, tents, luggage, handbags, shopping bags, airline headrests, CD-protection, pillowcases, and sandwich packaging.

Still another field of application of exemplary embodiment of the invention relates to school and office products. As examples, book covers, mailing envelopes, maps, signs and pennants, towels, and flags shall be mentioned.

Brief description of the drawings

The invention will be described in more detail hereinafter with reference to examples of embodiment but to which the invention is not limited:

Figure 1 illustrates a device for manufacturing nonwoven cellulose fiber fabric which is directly formed from lyocell spinning solution being coagulated by a coagulation fluid according to an exemplary embodiment of the invention.

Figure 2 to Figure 4 show experimentally captured images of nonwoven cellulose fiber fabric according to an exemplary embodiment of the invention in which merging of individual fibers has been accomplished by a specific process control.

Figure 5 and Figure 6 show experimentally captured images of nonwoven cellulose fiber fabric according to an exemplary embodiment of the invention in which swelling of fibers has been accomplished, wherein Figure 5 shows the fiber fabric in a dry non-swollen state and Figure 6 shows the fiber fabric in a humid swollen state.

Figure 7 shows an experimentally captured image of nonwoven cellulose fiber fabric according to an exemplary embodiment of the invention in which formation of two superposed layers of fibers has been accomplished by a specific process implementing two serial bars of nozzles.

Figure 8 shows a schematic image of a nonwoven cellulose fiber fabric according to still another exemplary embodiment of the invention composed of two stacked and merged layers of interconnected fibers having different average fiber diameter.

Figure 9 illustrates a part of a device for manufacturing nonwoven cellulose fiber fabric composed of two stacked layers of endless cellulose fiber webs according to an exemplary embodiment of the invention.

Figure 10 shows a schematic image of nonwoven cellulose fiber fabric according to an exemplary embodiment of the invention composed of three stacked layers with different average diameters of fibers.

Figure 11 shows a schematic image of nonwoven cellulose fiber fabric according to an exemplary embodiment of the invention composed of three stacked layers and configured as lotion delivery system with a marker visually indicating a user progress of active agent release.

Figure 12 shows how a roundness of fibers having a cross-section deviating from a circular cross-section can be calculated as a ratio between an inscribed circle and a circumscribed circle of the cross-section of the fiber according to an exemplary embodiment of the invention.

Figure 13 is a diagram illustrating optical gray value of a nonwoven cellulose fiber fabric according to an exemplary embodiment of the invention in a wet state.

Detailed description of the drawings

The illustrations in the drawings are schematic. In different drawings similar or identical elements are provided with the same reference labels.

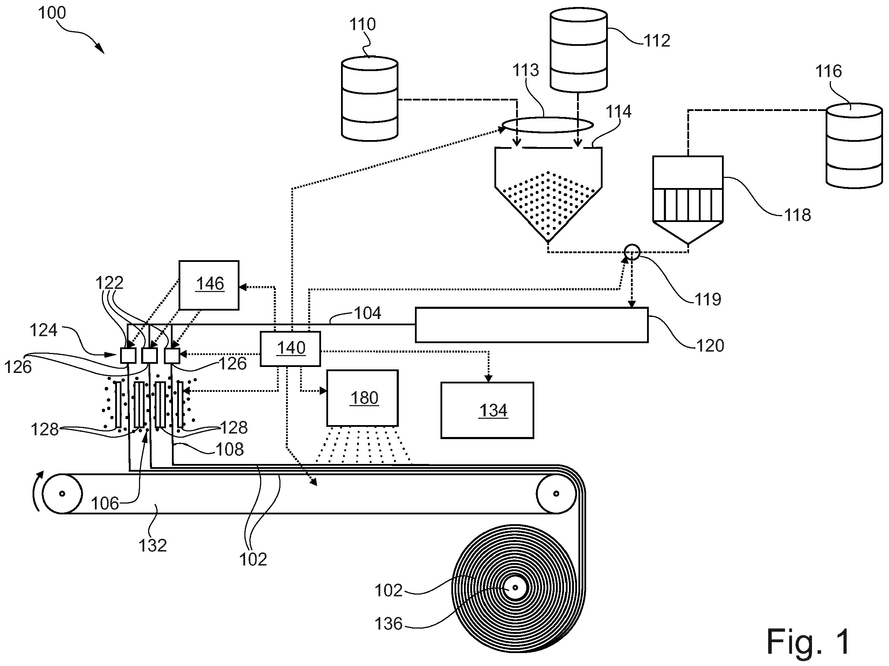

**Figure 1** illustrates a device 100 according to an exemplary embodiment of the invention for manufacturing nonwoven cellulose fiber fabric 102 which is directly formed from lyocell spinning solution 104. The latter is at least partly coagulated by a coagulation fluid 106 to be converted into partly-formed cellulose fibers 108. By the device 100, a lyocell solution blowing process according to an exemplary embodiment of the invention may be carried out. In the context of the present application, the term "lyocell solution-blowing process" may particularly encompass processes which can result in essentially endless filaments or fibers 108 of a discrete length or mixtures of endless filaments and fibers of discrete length being obtained. As further described below, nozzles each having an orifice 126 are provided through which cellulose solution or lyocell spinning solution 104 is ejected together with a gas stream or gas flow 146 for manufacturing the nonwoven cellulose fiber fabric 102 according to an exemplary embodiment of the invention.

As can be taken from Figure 1, wood pulp 110, other cellulose-based feedstock or the like may be supplied to a storage tank 114 via a metering unit 113. Water from a water container 112 is also supplied to the storage tank 114 via metering unit 113. Thus, the metering unit 113, under control of a control unit 140 described below in further detail, may define relative amounts of water and wood pulp 110 to be supplied to the storage tank 114. A solvent (such as N-methyl-morpholine, NMMO) accommodated in a solvent container 116 may be concentrated in a concentration unit 118 and may then be mixed with the mixture of water and wood pulp 110 or other cellulose-based feedstock with definable relative amounts in a mixing unit 119. Also the mixing unit 119 can be controlled by the control unit 140. Thereby, the water-wood pulp 110 medium is dissolved in the concentrated solvent in a dissolving unit 120 with adjustable relative amounts, thereby obtaining lyocell spinning solution 104. The aqueous lyocell spinning solution 104 can be a honey-viscous medium composed of (for instance 5 mass % to 15 mass %) cellulose comprising wood pulp 110 and (for instance 85 mass % to 95 mass %) solvent.

The lyocell spinning solution 104 is forwarded to a fiber formation unit 124 (which may be embodied as or which may comprise a number of spinning beams or jets 122). For instance, the number of orifices 126 of the jets 122 may be larger than 50, in particular larger than 100. In one embodiment, all orifices 126 of a fiber formation unit 124 (which may comprise a number of spinnerets or jets 122) of orifices 126 of the jets 122 may have the same size and/or shape. Alternatively, size and/or shape of different orifices 126 of one jet 122 and/or orifices 126 of different jets 122 (which may be arranged serially for forming a multilayer fabric) may be different.

When the lyocell spinning solution 104 passes through the orifices 126 of the jets 122, it is divided into a plurality of parallel strands of lyocell spinning solution 104. A vertically oriented gas flow, i.e. being oriented substantially parallel to spinning direction, forces the lyocell spinning solution 104 to transform into increasingly long and thin strands which can be adjusted by changing the process conditions under control of control unit 140. The gas flow may accelerate the lyocell spinning solution 104 along at least a part of its way from the orifices 126 to a fiber support unit 132.

While the lyocell spinning solution 104 moves through the jets 122 and further downward, the long and thin strands of the lyocell spinning solution 104 interact with non-solvent coagulation fluid 106. The coagulation fluid 106 is advantageously embodied as a vapor mist, for instance an aqueous mist. Process relevant properties of the coagulation fluid 106 are controlled by one or more coagulation units 128, providing the coagulation fluid 106 with adjustable properties. The coagulation units 128 are controlled, in turn, by control unit 140. Preferably, respective coagulation units 128 are provided between the individual nozzles or orifices 126 for individually adjusting properties of respective layers of fabric 102 being produced. Preferably, each jet 122 may have two assigned coagulation units 128, one from each side. The individual jets 122 can thus be provided with individual portions of lyocell spinning solution 104 which may also be adjusted to have different controllable properties of different layers of manufactured fabric 102.

When interacting with the coagulation fluid 106 (such as water), the solvent concentration of the lyocell spinning solution 104 is reduced, so that the cellulose of the former e.g. wood pulp 110 (or other feedstock) is at least partly coagulated as long and thin cellulose fibers 108 (which may still contain residual solvent and water).

During or after initial formation of the individual cellulose fibers 108 from the extruded lyocell spinning solution 104, the cellulose fibers 108 are deposited on fiber support unit 132, which is here embodied as a conveyor belt with a planar fiber accommodation surface. The cellulose fibers 108 form a nonwoven cellulose fiber fabric 102 (illustrated only schematically in Figure 1). The nonwoven cellulose fiber fabric 102 is composed of continuous and substantially endless filaments or fibers 108.

Although not shown in Figure 1, the solvent of the lyocell spinning solution 104 removed in coagulation by the coagulation unit 128 and in washing in a washing unit 180 can be at least partially recycled.

While being transported along the fiber support unit 132, the nonwoven cellulose fiber fabric 102 can be washed by washing unit 180 supplying wash liquor to remove residual solvent and may then be dried. It can be further processed by an optional but advantageous further processing unit 134. For instance, such a further processing may involve hydro-entanglement, needle punching, impregnation, steam treatment with a pressurized steam, calendering, etc.

The fiber support unit 132 may also transport the nonwoven cellulose fiber fabric 102 to a winder 136 on which the nonwoven cellulose fiber fabric 102 may be collected as a substantially endless sheet. The nonwoven cellulose fiber fabric 102 may then be shipped as roll-good to an entity manufacturing products such as wipes or textiles based on the nonwoven cellulose fiber fabric 102.

As indicated in Figure 1, the described process may be controlled by control unit 140 (such as a processor, part of a processor, or a plurality of processors). The control unit 140 is configured for controlling operation of the various units shown in Figure 1, in particular one or more of the metering unit 113, the mixing unit 119, the fiber formation unit 124, the coagulation unit(s) 128, the further processing unit 134, the dissolution unit 120, the washing unit 118, etc. Thus, the control unit 140 (for instance by executing computer executable program code, and/or by executing control commands defined by a user) may precisely and flexibly define the process parameters according to which the nonwoven cellulose fiber fabric 102 is manufactured. Design parameters in this context are air flow along the orifices 126, properties of the coagulation fluid 106, drive speed of the fiber support unit 132, composition, temperature and/or pressure of the lyocell spinning solution 104, etc. Additional design parameters which may be adjusted for adjusting the properties of the nonwoven cellulose fiber fabric 102 are number and/or mutual distance and/or geometric arrangement of the orifices 126, chemical composition and degree of concentration of the lyocell spinning solution 104, etc. Thereby, the properties of the nonwoven cellulose fiber fabric 102 may be properly adjusted, as described below. Such adjustable properties (see below detailed description) may involve one or more of the following properties: diameter and/or diameter distribution of the fibers 108, amount and/or regions of merging between fibers 108, a purity level of the fibers 108, properties of a multilayer fabric 102, optical properties of the fabric 102, fluid retention and/or fluid release properties of the fabric 102, mechanical stability of the fabric 102, smoothness of a surface of the fabric 102, cross-sectional shape of the fibers 108, etc.

Although not shown, each spinning jet 122 may comprise a polymer solution inlet via which the lyocell spinning solution 104 is supplied to the jet 122. Via an air inlet, a gas flow 146 can be applied to the lyocell spinning solution 104. Starting from an interaction chamber in an interior of the jet 122 and delimited by a jet casing, the lyocell spinning solution 104 moves or is accelerated (by the gas flow 146 pulling the lyocell spinning solution 104 downwardly) downwardly through a respective orifice 126 and is laterally narrowed under the influence of the gas flow 146 so that continuously tapering cellulose filaments or cellulose fibers 108 are formed when the lyocell spinning solution 104 moves downwardly together with the gas flow 146 in the environment of the coagulation fluid 106.

Thus, processes involved in the manufacturing method described by reference to Figure 1 may include that the lyocell spinning solution 104, which may also be denoted as cellulose solution is shaped to form liquid strands or latent filaments, which are drawn by the gas flow 146 and significantly decreased in diameter and increased in length. Partial coagulation of latent filaments or fibers 108 (or preforms thereof) by coagulation fluid 106 prior to or during web formation on the fiber support unit 132 may also be involved. The filaments or fibers 108 are formed into web like fabric 102, washed, dried and may be further processed (see further processing unit 134), as required. The filaments or fibers 108 may for instance be collected, for example on a rotating drum or belt, whereby a web is formed.

As a result of the described manufacturing process and in particular the choice of solvent used, the fibers 108 have a copper content of less than 5 ppm and have a nickel content of less than 2 ppm. This advantageously improves purity of the fabric 102.

The lyocell solution blown web (i.e. the nonwoven cellulose fiber fabric 102) according to exemplary embodiments of the invention preferably exhibits one or more of the following properties:

(i) The dry weight of the web is from 5 to 300 g/m$^2$, preferably 10-80 g/m$^2$

(ii) The thickness of the web according to the standard WSP120.6 respectively DIN29073 (in particular in the latest version as in force at the priority date of the present patent application) is from 0.05 to 10.0 mm, preferably 0.1 to 2.5 mm

(iii) The specific tenacity of the web in MD according to EN29073-3, respectively ISO9073-3 (in particular in the latest version as in force at the priority date of the present patent application) ranges from 0.1 to 3.0 $Nm^2/g$, preferably from 0.4 to 2.3 $Nm^2/g$

(iv) The average elongation of the web according to EN29073-3, respectively ISO9073-3 (in particular in the latest version as in force at the priority date of the present patent application) ranges from 0.5 to 100%, preferably from 4 to 50%.

(v) The MD/CD tenacity ratio of the web is from 1 to 12

(vi) The water retention of the web according to DIN 53814 (in particular in the latest version as in force at the priority date of the present patent application) is from 1 to 250%, preferably 30 to 150%

(vii) The water holding capacity of the web according to DIN 53923 (in particular in the latest version as in force at the priority date of the present patent application) ranges from 90 to 2000%, preferably 400 to 1100%.

(viii) Metal residue levels of copper content of less than 5 ppm and nickel content of less than 2 ppm, according to the standards EN 15587-2 for the substrate decomposition and EN 17294-2 for the ICP-MS analysis (in particular in the latest version as in force at the priority date of the present patent application)

Most preferably, the lyocell solution-blown web exhibits all of said properties (i) to (viii) mentioned above.

As described, the process to produce the nonwoven cellulose fiber fabric 102 preferably comprises:

(a) Extruding a solution comprising cellulose dissolved in NMMO (see reference numeral 104) through the orifices 126 of at least one jet 122, thereby forming filaments of lyocell spinning solution 104

(b) Stretching said filaments of lyocell spinning solution 104 by a gaseous stream (see reference numeral 146)

(c) Contacting said filaments with a vapor mist (see reference numeral 106), preferably containing water, thereby at least partly precipitating said fibers 108. Consequently, the filaments or fibers 108 are at least partly precipitated before forming web or nonwoven cellulose fiber fabric 102.

(d) Collecting and precipitating said filaments or fibers 108 in order to form a web or nonwoven cellulose fiber fabric 102

(e) Removing solvent in wash line (see washing unit 180)

(f) Optionally bonding via hydro-entanglement, needle punching, etc. (see further processing unit 134)

(g) Drying and roll collection

Constituents of the nonwoven cellulose fiber fabric 102 may be bonded by merging, intermingling, hydrogen bonding, physical bonding such as hydroentanglement or needle punching, and/or chemical bonding.

In order to be further processed, the nonwoven cellulose fiber fabric 102 may be combined with one or more layers of the same and/or other materials, such as (not shown) layers of synthetic polymers, cellulosic fluff pulp, nonwoven webs of cellulose or synthetic polymer fibers, bicomponent fibers, webs of cellulose pulp, such as airlaid or wetlaid pulp, webs or fabrics of high tenacity fibers, hydrophobic materials, high performance fibers (such as temperature resistant materials or flame retardant materials), layers imparting changed mechanical properties to the final products (such as Polypropylene or Polyester layers), biodegradable materials (e.g. films, fibers or webs from Polylactic acid), and/or high bulk materials.

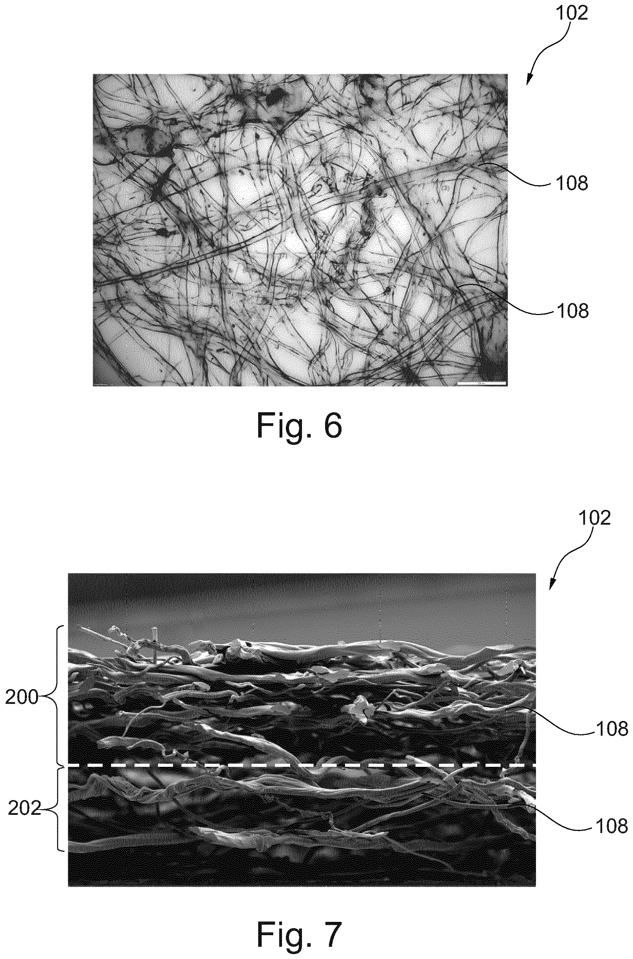

It is also possible to combine several distinguishable layers of nonwoven cellulose fiber fabric 102, see for instance Figure 7.

The nonwoven cellulose fiber fabric 102 may essentially consist of cellulose alone. Alternatively, the nonwoven cellulose fiber fabric 102 may comprise a mixture of cellulose and one or more other fiber materials. The nonwoven cellulose fiber fabric 102, furthermore, may comprise a bicomponent fiber material. The fiber material in the nonwoven cellulose fiber fabric 102 may at least partly comprise a modifying substance. The modifying substance may be selected from, for example, the group consisting of a polymeric resin, an inorganic resin, inorganic pigments, antibacterial products, nanoparticles, lotions, fire-retardant products, absorbency-improving additives, such as superabsorbent resins, ion-exchange resins, carbon compounds such as active carbon, graphite, carbon for electrical conductivity, X-ray contrast substances, luminescent pigments, and dye stuffs.

Concluding, the cellulose nonwoven web or nonwoven cellulose fiber fabric 102 manufactured directly from the lyocell spinning solution 104 allows access to value added web performance which is not possible via staple fiber route. This includes the possibility to form uniform lightweight webs, to manufacture microfiber products, and to manufacture continuous filaments or fibers 108 forming a web. Moreover, compared to webs from staple fibers, several manufacturing procedures are no longer required. Moreover, nonwoven cellulose fiber fabric 102 according to exemplary embodiments of the invention is biodegradable and manufactured from sustainably sourced raw material (i.e. wood pulp 110 or the like). Furthermore, it has advantages in terms of purity and absorbency. Beyond this, it has an adjustable mechanical strength, stiffness and softness. Furthermore, nonwoven cellulose fiber fabric 102 according to exemplary embodiments of the invention may be manufactured with low weight per area (for instance 10 to 30 g/m$^2$). Very fine filaments down to a diameter of not more than 5 $\mu$m, in particular not more than 3 $\mu$m, can be manufactured with this technology. Furthermore, nonwoven cellulose fiber fabric 102 according to an exemplary embodiment of the invention may be formed with a wide range of web aesthetics, for instance in a flat crispy film-like way, in a paper-like way, or in a soft flexible textile-like way. By adapting the process parameters of the described process, it is furthermore possible to precisely adjust stiffness and mechanical rigidity or flexibility and softness of the nonwoven cellulose fiber fabric 102. This can be adjusted for instance by adjusting a number of merging positions, the number of layers, or by after-treatment (such as needle punch, hydro-entanglement and/or calendering). It is in particular possible to manufacture the nonwoven cellulose fiber fabric 102 with a relatively low basis weight of down to 10 g/m$^2$ or lower, to obtain filaments or fibers 108 with a very small diameter (for instance of down to 3 to 5 $\mu$m, or less), etc.

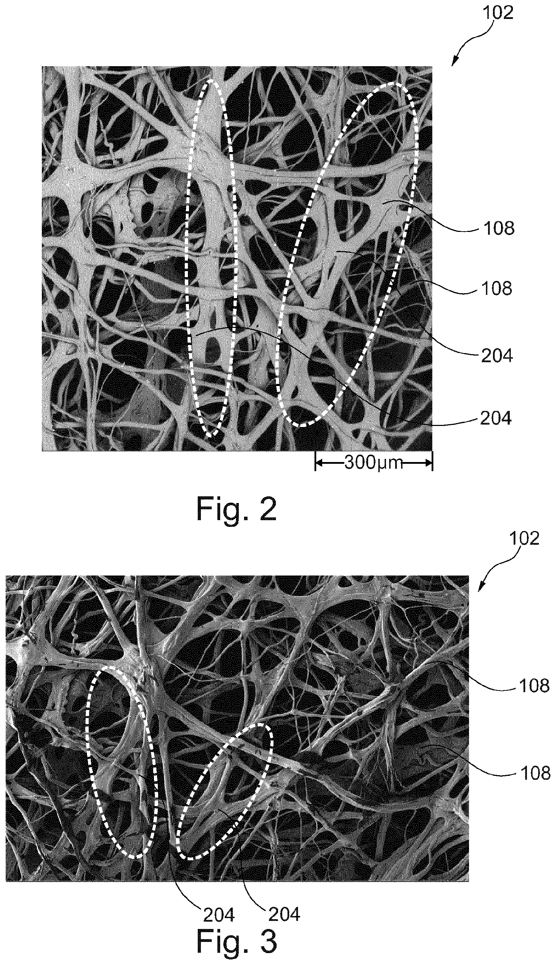

**Figure 2, Figure 3** and **Figure 4** show experimentally captured images of nonwoven cellulose fiber fabric 102 according to an exemplary embodiment of the invention in which merging of individual fibers 108 has been accomplished by a corresponding process control. The oval markers in Figure 2 to Figure 4 show such merging regions where multiple fibers 108 are integrally connected to one another. At such merging points, two or more fibers 108 may be interconnected to form an integral structure.

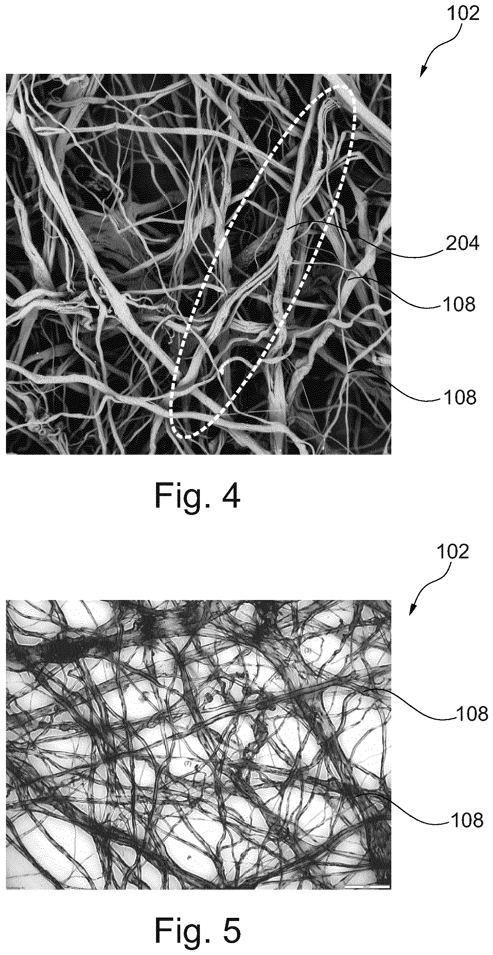

**Figure 5** and **Figure 6** show experimentally captured images of nonwoven cellulose fiber fabric 102 according to an exemplary embodiment of the invention in which swelling of fibers 108 has been accomplished, wherein Figure 5 shows the fiber fabric 102 in a dry non-swollen state and Figure 6 shows the fiber fabric 102 in a humid swollen state. The pore diameters can be measured in both states of Figure 5 and Figure 6 and can be compared to one another. When calculating an average value of 30 measurements, a decrease of the pore size by swelling of the fibers 108 in an aqueous medium up to 47% of their initial diameter could be determined.

**Figure 7** shows an experimentally captured image of nonwoven cellulose fiber fabric 102 according to an exemplary embodiment of the invention in which formation of two superposed layers 200, 202 of fibers 108 has been accomplished by a corresponding process design, i.e. a serial arrangement of multiple spinnerets. The two separate, but connected layers 200, 202 are indicated by a horizontal line in Figure 7. For instance, an n-layer fabric 102 (n≥2) can be manufactured by serially arranging n spinnerets or jets 122 along the machine direction.

Specific exemplary embodiments of the invention will be described in the following in more detail:

**Figure 8** shows a schematic cross sectional view of a nonwoven cellulose fiber fabric 102 according to an exemplary embodiment of the invention composed of two stacked and merged layers 200, 202 of interconnected fibers 108 having different fiber thicknesses d and D>d (see the lower two details of Figure 8). More specifically, different ones of the fibers 108 being located in the different layers 200, 202 differ concerning an averaged fiber diameter (i.e. averaged over the fibers 108 of the respective layer 200, 202). Fibers 108 of the respective layers 200, 202 are also merged at merging positions 204, compare the lower two details of Figure 8. A further detail of the interface between the layers 200, 202 is shown as well, where a merging point 204 is visible which integrally couples fibers 108 of both layers 200, 202 at the interface for increasing stability of the fabric 102 at the interface (see the upper detail of Figure 8). Additionally, different ones of the fibers 108 being located in the different layers 200, 202 are integrally connected at at least one respective merging position 204.

Merging properties may be adjusted to obtain desired properties. For instance, a number of merging points 204 per volume of fabric 102 may be adjusted separately within the respective one of the layers 200, 202 and/or between the layers 200, 202. This can be done by adjusting the coagulation properties (in particular coagulation of filaments of lyocell spinning solution 104 upstream of the fiber accommodation surface of the fiber support unit 132, coagulation of filaments of lyocell spinning solution 104 after lay down of the filaments on the fiber accommodation surface of the fiber support unit 132, etc.). The merging between the different layers 200, 202 may be adjusted so that pulling on the layers 200, 202 in opposite directions results in a separation of the fabric 102 at an interface between the different layers 200, 202. In other words, a merging-based connection force between the different layers 200, 202 may be adjusted to be smaller than a merging based connection force within a respective one of the different layers 200, 202.

The fibers 108 located in the different layers 200, 202 and being formed with different average diameter and different merging properties may be provided with different functionalities. Such different functionalities may be supported by the different average diameters, but may also be further promoted by a respective coating or the like. Such different functionalities may for instance be a different behavior in terms of wicking, anisotropic behavior, different oil holding capability, different water absorbing and holding capability, different cleanability, different mechanical properties and/or different roughness.

The mentioned functionalization may also involve an adaptation of the manufactured nonwoven cellulose fiber fabric 102 as an optical switch which can be transformed between a dry optically opaque state and a wet optically transparent state by the mere supply of water, an aqueous solution, oil, etc., or a liquid removal procedure (for instance drying the fabric 102 by evaporating liquid therein by heating). Since the fabric 102 can be manufactured in a very pure way, i.e. consisting substantially of cellulose with low contamination with impurities, the optical transparency in the wet state is quite pronounced. By promoting liquid absorbing capability and by adjusting a high wicking speed, a high optical transparency in the liquid soaked state of the fabric 102 and a quick switch between different light transmissivity conditions of the fabric 102 can be accomplished. Process parameters which can be adjusted for that purpose are for instance adjusting a high degree of roundness of the fibers 108, accomplishing *inter-fiber* and *intra*-fiber interconnection by integrally forming cellulose merging positions 204, suppressing impurities (in particular heavy metal impurities) of the fabric 102, etc. For instance, it is also possible that the process parameters of the manufacturing process of producing fabric 102 shown in Figure 8 are adjusted so that the endless fibers 108 have an amount of fiber ends per volume of not more than 5,000 ends/cm$^3$ in a fabric having a density of 0.1 t/m$^3$. Since also free fiber ends in an interior of fabric 102 may serve as scattering centers for light, the strong reduction of the amount of such free fiber ends (in particular compared with staple fibers) further promotes optical transmissivity in the wet state of the fabric 102. For instance, the process parameters during manufacturing the fabric 102 may be adjusted so that a wicking speed is at least 0.025 g/s. Thus, liquid such as water may rapidly enter fabric 102, may rapidly spread across fiber 102, and may also be quickly removed therefrom (in terms of a drying procedure, which may be promoted by heating fabric 102 to an elevated temperature).

The multilayer nonwoven cellulose fiber fabric 102 according to Figure 8 can be directly manufactured from lyocell spinning solution 104 using the device 100 and corresponding manufacturing method described below referring to Figure 9. Advantageously, the partial heavy metal contaminations of the fibers 108 of the fabric 102 according to Figure 8 are not more than 10 ppm for each individual chemical heavy metal element (i.e. not more than 10 ppm for iron, not more than 10 ppm for zinc, not more than 10 ppm for cadmium, etc.). Beyond this, an overall or entire heavy metals content of fabric 102 summed up for all heavy metal chemical elements together (i.e. in particular for Cr, Mn, Fe, Co, Ni, Cu, Zn, Zr, Mo, Cd, Sn, W, Pb, Bi) is not more than 30 ppm. Apart from this, the fibers 108 have a copper content of less than 5 ppm and have a nickel content of less than 2 ppm. This is a consequence of the operating fluids (in particular lyocell spinning solution 104, coagulation fluid 106, washing liquor, gas flow 146, etc.) which are used during the manufacturing process and which may be substantially free of heavy metal sources such as copper salt. As a result of this design of the manufacturing process, the fibers 108 may be of high quality. The absence of any mentionable heavy metal impurities in the manufacturing process prevents highly undesired decomposition of involved media (in particular of the lyocell spinning solution 104) and therefore allows to obtain highly reproducible and highly pure cellulose fabric 102.

As a result of the above described manufacturing method and the corresponding properties of the fabric 102, the fabric 102 is optically transparent when being loaded with liquid, but can be converted into an optically opaque state by drying (i.e. by removing liquid from an interior of the fabric). This procedure is reversible and can be repeated multiple times. More specifically, the fabric 102 may be provided with an optical gray value of at least 90 (i.e. 90 or above), or even at least 100, when wet. In the opaque dry state of the fabric 102, the interior of the fabric 102 is substantially free of liquid such as water. In the opaque dry state of the fabric 102, the gray value may be below 85, in particular below 80. In other words, the fabric may be optically opaque (in particular with an optical gray value of lower than 85) when completely dry. The mentioned gray values corresponding to a scale from 0 to 255 and are measured in transmission geometry. In the optically transparent wet state of the fabric 102, a liquid such as water has entered into the fibers 108 (which results in fiber swelling) as well as in gaps between the fibers 108 in an interior of the fabric 100. In particular, the fabric 102 may be rendered optically transparent when being wet to such a degree that a mass ratio between a mass of moisture in an interior of the fabric 102 and a mass of the dry fibers 108 is above 3.

**Figure 9** illustrates a part of a device 100 for manufacturing nonwoven cellulose fiber fabric 102 composed of two stacked layers 200, 202 of endless cellulose fibers 108 according to an exemplary embodiment of the invention. A difference between the device 100 shown in Figure 9 and the device 100 shown in Figure 1 is that the device 100 according to Figure 9 comprises two serially aligned jets 122 and respectively assigned coagulation units 128, as described above. In view of the movable fiber accommodation surface of the conveyor belt-type fiber support unit 132, the upstream jet 122 on the left-hand side of Figure 9 produces layer 202. Layer 200 is produced by the downstream jet 122 (see right hand side of Figure 9) and is attached to an upper main surface of the previously formed layer 202 so that a double layer 200, 202 of fabric 102 is obtained.

According to Figure 9, the control unit 140 (controlling the jets 122 and the coagulation units 128) is configured for adjusting process parameters so that the fibers 108 of the different layers 200, 202 differ concerning fiber diameter by more than 50% in relation to a smallest diameter (see for example Figure 8). Adjusting the fiber diameters of the fibers 108 of the layers 200, 202 by the control unit 140 may comprise adjusting an amount of coagulation fluid 106 interacting with the lyocell spinning solution 104. Additionally, the embodiment of Figure 9 adjusts the process parameters for adjusting fiber diameter by serially arranging multiple jets 122 with orifices 126 (optionally with different properties) along the movable fiber support unit 132. For instance, such different properties may be different orifice 126 diameters, different speed of gas flow 146, different amounts of gas flow 146, and/or different gas flow 146 pressure. Although not shown in Figure 9, it is possible to further process the fibers 108 after collection on the fiber support unit 132 by hydroentangling, needling, and/or impregnating.

In particular, the device 100 shown in Figure 9, when compared to the device 100 shown in Figure 1, comprises a further jet 122 with orifices 126 configured for extruding further lyocell spinning solution 104 supported by a further gas flow 146. As can be taken from Figure 9, the further jet 122 is arranged downstream of the jet 122. The jet 122 is configured for forming one of the layers 202, and the further jet 122 is configured for forming another one of the layers 200 upon the layer 202. The geometry shown in Figure 9 allows to freely and independently adjust the properties of the fibers 108 and the corresponding layer 200, 202, also in terms of adjusting its optical properties. Hence, one or more further nozzle bars or jets 122 may be provided and may be arranged serially along a transport direction of fiber support unit 132. The multiple jets 122 may be arranged so that further layer 200 of fibers 108 may be deposited on top of the previously formed layer 202, preferably before the coagulation or curing process of the fibers 108 of the layer 202 and/or of the layer 200 is fully completed, which may trigger merging. When properly adjusting the process parameters, this may have advantageous effects in terms of the properties of a multilayer fabric 102:

The device 100 according to Figure 9, which is configured for the manufacture of multilayer fabric 102, implements a high number of process parameters which can be used for designing optically relevant properties of the fibers 108 as well as of fiber layers 200, 202. This is the result of the serial arrangement of multiple jets 122, each of which being operable with individually adjustable process parameters.

With device 100 according to Figure 9, it is in particular possible to manufacture a fabric 102 composed of at least two layers 200, 202 (preferably more than two layers). The fibers 108 of the different layers 200, 202 may have different values of average diameter and may be formed in one continuous process. By taking this measure, a highly efficient production of the nonwoven cellulose fiber fabric 102 can be ensured, which in particular allows to transfer the obtained multilayer fabric 102 in one transport procedure to a destination for further processing.

By the defined layer separation of a multilayer fabric 102, it is also possible to later separate the multilayer fabric 102 into the different individual layers 200, 202 or into different multilayer sections. According to exemplary embodiments of the invention, both *intra*-layer adhesion of the fibers 108 of one layer 200, 202 as well as *inter*-layer adhesion of the fibers 108 between adjacent layers 200, 202 (for instance by merging and/or by friction generating contact) may be properly and individually adjusted. A corresponding separate control for each layer 200, 202 individually may be in particular obtained when the process parameters are adjusted so that coagulation or curing of the fibers 108 of one layer 202 is already completed when the other layer 200 of fibers 108 is placed on top thereof. All this can be obtained for a fabric 102 having a very low heavy metals content due to the adjusted lack of heavy metal sources along the process line.

**Figure 10** shows a schematic image of nonwoven cellulose fiber fabric 102 according to another exemplary embodiment of the invention composed of three stacked layers 202, 200, 200 with different diameters of fibers 108. According to Figure 10, an intermediate sandwich layer 200 has significantly smaller diameters of fibers 108 than the two exterior layers 200, 202 above and below.

The multilayer fabric 102 shown in Figure 10 is particularly appropriate for applications such as medical appliances, agricultural textiles, cosmetic application, etc. For instance, an active substance or a lotion may be stored in the inner layer 200 showing a high capillary action. The exterior layers 200, 202 may be designed in terms of rigidity and surface haptic. This is advantageous for cleaning and medical applications. For agricultural applications, the fiber layer design may be specifically configured in terms of evaporation properties and/or root penetration.

In another application, the multilayer fabric 102 shown in Figure 10 may be used as facial mask, industrial wipe, etc., wherein the central layer 200 may have a specifically pronounced fluid retaining capability. The cover layers 200, 202 may be configured for adjusting fluid release properties. The average diameters of the fibers 108 of the respective layer 200, 200, 202 may be used as a design parameter for adjusting these functions. In particular, the multilayer fabric 102 shown in Figure 10 may be configured as a lotion delivery system.

As mentioned above, an exemplary embodiment of the invention provides a nonwoven cellulose fiber fabric 102 with a very low contamination with heavy metal elements. This is promoted on the one hand by the above described configuration of lyocell spinning solution 104 and other media used along the production line which are by themselves substantially heavy metal element free. Simultaneously, also the hardware configuration of the device 100 may be configured so that substantially no re-contamination of the processed lyocell spinning solution 104 and the manufactured fibers 108 with heavy metal impurities occurs along the line. Thus, a biocompatible and biodegradable nonwoven cellulose fiber fabric 102 may be obtained.

In particular, also integral interconnection of fibers 108 of the fabric 102 by the formation of merging points 204 on the basis of lyocell spinning solution 104 (rather than by a separate adhesive or binder made of one or more additional materials) contributes significantly to the purity of the manufactured fabric 102. Thus, no highly disturbing heavy metals comprising connection points of separate adhesive or binder material need to be formed as a result of the process flow described referring to Figure 1 and Figure 9. The formation of merging positions 204 between fibers 108 of the fabric 102 can be accomplished by merely bringing filaments of lyocell spinning solution 104 in direct physical contact with one another prior to coagulation, i.e. before precipitation of solid fibers 108. This allows to obtain pure cellulose fabric 102 without additional adhesive material, with a precisely adjustable (in particular a strong) *inter*-fiber connection, with a moderate bulk density, and with very low residual amount of heavy metal elements and compounds. Thereby, a fabric 102 can be obtained which advantageously has a low environmental impact and which is not harmful to health for a user.

By the described cellulose filament production on the basis of lyocell spinning solution 104 it can be ensured that no production related heavy metal impurities accumulate in the manufactured fabric 102. This is particularly advantageous for post processing of such fabric 102 and when a correspondingly manufactured product gets into contact with human beings or natural organisms. The opportunity to manufacture nonwoven cellulose fiber fabric 102 with low heavy metal content (in particular low copper content) by a corresponding process control allows to prevent copper-based inhibiting or even toxic effects on microorganisms. Moreover, toxicity of copper may be reinforced by other heavy metals such as Hg, Sn, Cd. Thus, not only the low copper content, but also the low entire or overall heavy metal content of the fabric 102 manufactured with the above described manufacturing method is advantageous.

Moreover, wherein biodegradable nonwoven cellulose fiber fabric 102 decomposes after use, non-biodegradable heavy metal content thereof will not decompose and will therefore accumulate. Thus, fabric 102 according to an exemplary embodiment of the invention being poor in terms of heavy metal content is particularly appropriate for biodegradation after use without mentionable ecological footprint.

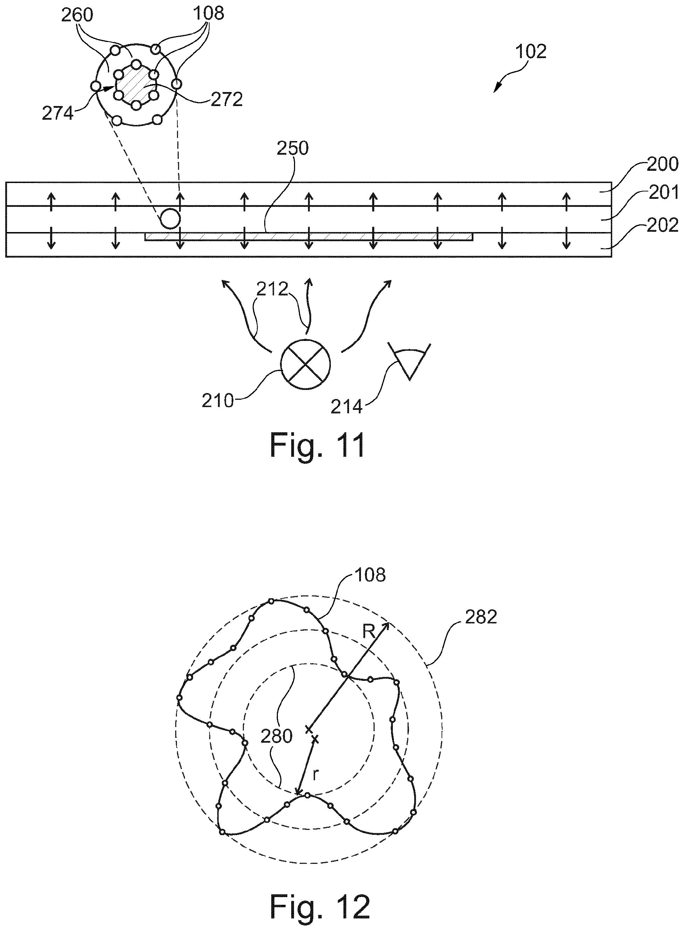

**Figure 11** shows a schematic image of nonwoven cellulose fiber fabric 102 according to an exemplary embodiment of the invention composed of three stacked layers 200, 201, 202 and configured as lotion delivery system. For instance, the fabric 102 of Figure 11 may be configured for delivering a cosmetic or medical lotion.

The product shown in a cross-sectional view of Figure 11 and being manufactured on the basis of a fabric 102 is composed of here exactly three interconnected layers 200, 201, 202. Between two opposing liquid permeable (in view of pores defined between the networked fibers 108) cover layers 200, 202, an intermediate layer 201 is sandwiched and embedded. An active agent 272 (such as a pharmaceutically active agent or a cosmetically active agent) is accommodated and retained in the intermediate layer 201. The active agent 272 is accommodated in cavities 274 (only one is shown) of the fiber network of layer 201 between several fibers 108 and may be held or retained in the tiny cavity 274 under the influence of capillary forces. The cavity 274 is in fluid communication with pores 260 which are also delimited between fibers 108 and serve as fluidic channels or conduits within the fabric 102. For instance triggered by a mechanical impact (such as squeezing of the fabric 102 shown in Figure 11 by applying a manual pressing force on the fabric 102 by a user), the active agent 272 can be released from the cavities 274 in the intermediate layer 201. From there, the active agent 272 can be released from the central intermediate layer 201 via a respective one of the cover layers 200, 202 towards an environment. Such an environment may for instance be the face skin of a user (not shown) onto which the fabric 102 may be attached, for instance if the shown product is a face mask.

Advantageously, the fabric 102 according to Figure 11 may be provided with a permanently opaque marker 250. In the shown embodiment, the marker 250 may be printed on an interface surface of layer 201 or layer 202. Alternatively, the marker at 250 may also be printed on an exterior surface of the fabric 102, preferably a surface of the fabric 102 attached to the destination of the active agent 272 (for instance a face of a user). The marker 250 is optically visible from an exterior of the fabric 102 when the fabric 102 is optically transparent, as indicated in Figure 11 showing a light source 210 emitting light 212 being reflected partially by the fabric 102 so as to be visible by a user's eye 214. When the active agent 272 is released from the intermediate layer 201 towards the face skin of the user, a moisture content of the fabric 102 is continuously reduced so that the fabric 102 turns from the wet optically transparent state into a dry optically opaque state. The latter occurs when the moisture content of the fabric 102 falls below a predetermined threshold value since the fabric 102 has dried out due to the continued release of the active agent 272. When the fabric 102 turns into the opaque state, the marker 250 may be no longer visible for the user, since the light 212 is no longer capable of propagating up to the marker 250. When the marker 250 provides a corresponding instruction to a user (such as "release of active agent is not yet completed - do not yet remove fabric"), loss of visibility of the marker 250 indicates to the user that the fabric 102 may now be removed from the face skin.

Reference is now made to Figure 12. Preferably, at least 80% of the fibers 108 have a cross sectional shape having a roundness of more than 90%. Thus, it is preferred for a high optical transmissivity in the wet state of fiber fabric 102 that the fibers 108 are as round as possible, i.e. ideally assume a circular cylindrical shape. This corresponds to a circular cross-section of the fibers 108. It is believed that deviations from the circular cross-sectional shape act as optical irregularities, promote undesired scattering of visible electromagnetic radiation and therefore deteriorate optical transmissivity of the fabric 102 in the wet state. For this reason, it is advantageous when the process parameters of the manufacturing method of manufacturing fabric 102 are adjusted so that the deviation of the cross section of the fibers from a circular cross-section is as small as possible. This can for instance be promoted by truly circular orifices 126, an adjusted gas flow 146 around filaments of lyocell spinning solution 104, adjusted coagulation conditions, a homogeneous viscosity of the lyocell spinning solution 104, etc.

**Figure 12** shows how a value of roundness of fibers 108 having a cross-section deviating from a circular cross-section can be calculated as the ratio between an inscribed circle 280 and a circumscribed circle 282 of the cross-section of the fiber 108 according to an exemplary embodiment of the invention.

The minimum circumscribed circle 282 is defined as the smallest circle which encloses whole of the roundness profile of the cross-section of the fiber 108 illustrated in Figure 12. The maximum inscribed circle 280 is defined as the largest circle that can be inscribed inside the roundness profile of the cross-section of the fiber 108 illustrated in Figure 12. In the context of the present application, roundness can be defined as a ratio between a radius r of the inscribed circle 280 divided by a radius R of the circumscribed surface 282. Roundness may be indicated by a resulting percentage value. In the present example, $R \approx 2r$ and the roundness of the fiber 108 is therefore approximately 0.5 or 50%. For comparison, a circular cylindrical fiber 108 fulfills the condition $R=r$ and has a roundness of one or 100%.

**Figure 13** is a diagram 290 illustrating optical gray value of a nonwoven cellulose fiber fabric 102 according to an exemplary embodiment of the invention in a wet state. The diagram 290 has an abscissa 292 along which gray values are plotted from 0 to 255. Furthermore, the diagram 290 has an ordinate 294 indicating a respective number of pixels per gray value.

The characteristic shown in diagram 290 and being indicative of an optical transparency of the investigated fabric 102 has been experimentally obtained by analyzing a fabric 102 material with a grammage of 38 $g/m^2$ which has been investigated for its wet transparency.

The test method was as follows. The wetted sample (10 fold loaded with water, equilibrium time 10 min) was put on an optically transparent plastic foil and on a defined light source with light shining through the sample. With software analysis the gray value of each pixel was measured.

The gray value at the received maximum pixel number is a value for transparency, respective opacity of the fabric. The higher the gray value the higher the transparency.

The instruments and conditions used were:

- Camera: Olympus Color View 2 BW 1040x772= (802880 Pixel)
- Light source: Volpi Intralux 6000-1
- Lens: Pentax 12mm
- Cold light board: Fostec
- Software: Olympus Analysis auto
- Shutter speed: 20ms
- Aperture: 4
- Picture width: 90.5mm

According to an exemplary embodiment of the invention, an optically transparent multilayer nonwoven cellulose fiber fabric 102 is provided. The optical transmissivity or translucency is high in a wet state of the fabric 102. Such a high wet transmissivity or translucency can be obtained by manufacturing highly pure cellulose fabric 102 of endless fibers 108 with a purely cellulose-based integral merging between different layers 200, 202. In order to manufacture such a fabric 102, it is possible to use a nozzle bar for generating filaments of lyocell spinning solution 104, which filaments are then stretched and laid down on a fiber support unit 132. The formation of fiber-to-fiber adhering merging positions 204 may be promoted by gas turbulence during the stretching procedure (so that the filaments get into physically contact prior to coagulation or precipitation of fibers 108) and/or during laydown on the fiber support unit 132. By providing at least one additional nozzle bar or jet 122 (compare Figure 9), a further layer 200 of fibers 108 may be laid down on the previously formed layer 202 of fibers 108, preferably before the fibers 108 of at least one of the interconnected layers 200, 202 had already completed coagulation and precipitation. Thereby, integral merging positions 204 or merging points of cellulose are formed interconnecting fibers 108 within the respective layer 200, 202 and between different layers 200, 202. This procedure at the same time may allow to maintain a proper separation between the different layers 200, 202. The so manufactured fabric 102 shows a pronounced optical transparency in a wet or moisture filled condition of the fabric 102. A proper adjustment of the process parameters of such a manufacturing process allows to prevent impurities to be introduced in the fabric 102 and may prevent optically less favored cross-sectional shapes of the fibers 108 (i.e. to promote a circular round cross-sectional shape rather than a flat or irregular cross-section of the fibers 108).

The fact that the manufacturing process described referring to Figure 1 and Figure 9 allows to obtain a fabric 102 being substantially free of heavy metal contents and other impurities promotes the proper optical transmission characteristic of the wet fabric 102. This also promotes a homogeneous intrinsic construction of the fibers 108 having an additionally positive impact on the optical transparency in the wet condition. A corresponding product is moreover biodegradable and biocompatible, in particular appropriate for being brought in contact with human beings and other natural organisms.

Surprisingly, the nonwoven cellulose fiber fabric 102 according to an exemplary embodiment is optically transparent in the wet state despite of the multilayer configuration. In particular an interface plane between different layers 200, 202 of such a fabric 102 is in general a source of undesired light scattering and optical diffusing. Integral merging of the layers 200, 202 rather than interconnecting them with a separate adhesive glue or the like provides a substantially uniform and homogeneous fabric 102 with nevertheless visually distinguishable and separately configurable layers 200, 202. Hence, merging can be controlled by controlling timing of laydown of the filaments or fibers 108 and/or of the various layers 200, 202 on top of each other, preferably before completing coagulation. By appropriate timing parameters, a similar filament coupling may be obtained between the distinguishable layers 200, 202 as within a respective layer 200, 202.

By the use of endless fibers 108 it can be ensured that only a minor number of free fiber ends (as occur in a high number in staple fibers) is present within the fabric 102. This increases the optical homogeneity and provides a larger optical transparency in the wet state.

Heavy metal additives have the capability of reducing optical transparency already in very small amounts. For instance, a heavy metal compound comprising cobalt may already cause a blue color in a concentration significantly below 100 ppm. By manufacturing the fibers 108 substantially without heavy metal contamination, the optical transparency in the wet state may be further promoted.

A multi-layer multi-functionality fabric 102 according to an exemplary embodiment of the invention may result from a separate functionalization of different layers 200, 202. The fabric 102 made of endless cellulose fibers 108 offers a particularly high number of material properties and geometric properties which can be used for adjusting the fabric 102 to obtain a certain function.

In an exemplary embodiment, a fast reacting liquid display system can be provided (for instance for cosmetic applications or the like), which allows to distinguish visually clearly and unambiguously between "wet" and "dry". Simultaneously, such a reaction can be obtained very fast. This is for instance of high advantage for applications such as splash masks, i.e. face masks having a short application time. By the use of cellulose fibers 108, the hydrophilic property of the cellulose material may accelerate effects connected with capillary forces, in particular fast wetting characteristic and pronounced wicking speed. This results in a quick accommodation of humidity in the fabric 102, which, in turn, results in a quick formation of a transparent state. Moreover, the use of endless cellulose fibers 108 allows to obtain an improved transparency for light in the visible range when the respective nonwoven cellulose fiber fabric 102 is brought in interaction with moisture. As compared to staple fibers, endless fibers 108 do not suffer significantly from disturbing fiber transitions and free fiber ends.

Under consideration of the refraction index of the (substantially colorless) cellulose of about 1.47 to 1.49 (depending on the frequency) as compared to a refraction index of water of about 1.33, the number of transitions between cellulose material and water in the wet fabric 102 should be kept as small as possible to obtain a high optical transmissivity. Each transition can cause undesired diffusion or refraction of light which reduces the transmitted light intensity. It has turned out that the formation of the fibers 108 with a cross-sectional shape being exactly or at least approximately circular results in a lower loss of light energy compared to flat or irregular cross-sections of fibers 108.

In yet another exemplary embodiment (which can be used advantageously for agricultural applications), endless cellulose fibers 108 may be used for accomplishing a fast transport of moisture along a fiber 108. In the framework of a fabric 102 composed of multiple of such endless fibers 108, this results in a very rapid liquid spreading along a two-dimensional area. In addition, the use of endless cellulose fibers 108 allows to obtain an improved transparency for light in the visible range when the fabric 102 is humidified. In view of this rapid and efficient response of the transparency in the presence of moisture, it is possible to create self-controlled biological systems. Agricultural issues such as undesired drying can be inspected automatically using a fabric 102 according to an exemplary embodiment of the invention. Since the fabric 102 turns from an optically transparent condition into an opaque condition when an excessive amount of moisture is released from the fabric 102, a need of additional water can be detected optically by inspecting the fabric 102, and new moisture can be delivered to the fabric 102 if a change of the optical transmissivity is detected.

In yet another exemplary embodiment, a fabric 102 may be used also for the agricultural application described in the following: sun light activates color pigments in fruits which thereby change color. In particular, the sun induced enzymatic decomposition of tanning agent and fruit acids may be influenced by adjusting transmissivity of a fabric 102 covering fruits or plants. Therefore, humidity may be supplied to such a fabric 102 for switching sunlight on for a fruit or plant. By properly positioning such a fabric 102 also sunlight reflection properties may be controlled. In case of "wet", the optical transmissivity of the fabric 102 may direct the sunlight into the ground. In case of "dry", the opaque property of the fabric 102 may reflect the sunlight to thereby influence maturation processes. For the example of fruits, maturation by light may be controlled in such a way that at high irradiation power, compounds in the fluid being instable with respect to light and oxygen can be decomposed for supporting the maturation process. Further functional mechanisms which can be influenced by taking this measure are related to photo-oxidation.

A nonwoven cellulose fiber fabric 102 according to an exemplary embodiment of the invention may be also used for the following cosmetic application related for instance to face masks, more particularly to splash masks. For such applications, a quickly reacting liquid display system is desired which visually distinguishes clearly and unambiguously between dry and wet. In particular when using endless cellulose fibers 108 with a diameter range between 5 $\mu$m

and 20 $\mu$m, a thin sheet like nonwoven cellulose fiber fabric 102 can be manufactured which nevertheless has a high retention capability for a liquid and provides for a high delivery rate of an active agent 274. By this thin geometry in combination with a high liquid storage capability, it is possible to optically visualize the different operation states "wet" and "dry" of the face mask. In particular, on the backside of the foil of fabric 102 a text such as "Please wait, active agent is released" may be printed as a marker 250 which is no longer readable when the fabric 102 has dried (since the fabric 102 then turns opaque).

Summarizing, in particular one or more of the following adjustments may be made according to exemplary embodiments of the invention:

- a low homogeneous fiber diameter may allow to obtain a high smoothness of the fabric 102
- multilayer fabric 102 with low fiber diameter may allow to obtain a high fabric thickness at a low fabric density
- equal absorption curves of the functionalized layers can allow to obtain a homogeneous humidity and fluid accommodation behavior, as well as a homogenous behavior in terms of fluid release
- the described connection of layers 200, 202 of fabric 102 allows to design products with low linting upon layer separation
- it is also possible to differently functionalize single layers 200, 202 so that products with anisotropic properties are obtained (for instance for wicking, oil accommodation, water accommodation, cleanability, roughness).

Finally, it should be noted that the above-mentioned embodiments illustrate rather than limit the invention, and that those skilled in the art will be capable of designing many alternative embodiments without departing from the scope of the invention as defined by the appended claims. In the claims, any reference signs placed in parentheses shall not be construed as limiting the claims. The words "comprising" and "comprises", and the like, do not exclude the presence of elements or steps other than those listed in any claim or the specification as a whole. The singular reference of an element does not exclude the plural reference of such elements and vice-versa. In a device claim enumerating several means, several of these means may be embodied by one and the same item of software or hardware. The mere fact that certain measures are recited in mutually different dependent claims does not indicate that a combination of these measures cannot be used to advantage.

## Claims

1. A nonwoven cellulose fiber fabric (102), in particular directly manufactured from lyocell spinning solution (104), wherein the fabric (102) comprises a network of substantially endless fibers (108), wherein different ones of the fibers (108) are located at least partially in different distinguishable interconnected layers (200, 202), and wherein the fabric (102) is optically transparent when wet.

2. The fabric (102) according to claim 1, wherein the fabric (102) is optically opaque, in particular with an optical gray value of lower than 85, when completely dry.

3. The fabric (102) according to claim 1 or 2, wherein the fabric (102) has an optical gray value of at least 90, in particular of at least 100, when wet.

4. The fabric (102) according to any of claims 1 to 3, wherein the fabric (102) is optically transparent when being wet to such a degree that a mass ratio between a mass of moisture, in particular water, in an interior of the fabric (102) and a mass of the fibers (108) is at least 3, in particular is at least 5, more particularly is at least 7.

5. The fabric (102) according to any of claims 1 to 4, comprising at least one of the following features:

   wherein fibers (108) of a respective layer (200, 202) are integrally merged at at least one merging position (204) within said layer (200, 202);
   wherein fibers (108) of different layers (200, 202) are integrally merged at at least one merging position (204) between said layers (200, 202).

6. The fabric (102) according to claim 5, comprising at least one of the following features:

   wherein the merging between the different layers (200, 202) is adjusted so that pulling on the layers (200, 202) in opposite directions results in a separation of the fabric (102) at an interface between the different layers (200, 202);

wherein the merging is adjusted so that a merging-based connection force between the different layers (200, 202) is smaller than a merging based connection force within a respective one of the different layers (200, 202).

7. The fabric (102) according to any of claims 1 to 6, comprising a permanently opaque marker (250) being optically visible through at least a part of the fabric (102) when a moisture content of the fabric (102) is above a predetermined threshold value and being optically invisible through at least a part of the fabric (102) when the moisture content of the fabric (102) is below the predetermined threshold value, in particular when the fabric (102) is dry.

8. The fabric (102) according to any of claims 1 to 7, comprising at least one of the following features:

comprising at least three interconnected layers (200, 201, 202) composed at least of two opposing cover layers (200, 202) between which an intermediate layer (201) is embedded, wherein an active agent (272) is accommodated in the intermediate layer (201) and is releasable via at least one of the cover layers (200, 202) towards an environment;
wherein an adhesion force between the distinguishable interconnected layers (200, 202) is smaller than an adhesion force within a respective one of the layers (200, 202);
wherein an average diameter (D, d) of the fibers (108) of a respective layer (200, 202) is different from an average diameter (D, d) of the fibers (108) of a respective other layer (200, 202);
wherein an interconnection between the layers (200, 202) is accomplished without separate binder or glue material;
wherein an interconnection between fibers (108) within a respective one of the layers (200, 202) is accomplished without separate binder or glue material;
wherein the endless fibers (108) have an amount of fiber ends per volume of less than 10,000 ends/cm$^3$, in particular less than 5,000 ends/cm$^3$;
wherein at least 50%, in particular at least 80%, of the fibers (108) have a cross sectional shape having a roundness of more than 60%, in particular of more than 80%;
wherein the fabric (102) is configured so that a wicking speed is at least 0.25 g water / g fabric/s;
wherein at least 80 mass percent of the fibers (108) have an average fiber diameter (D, d) in a range between 1 $\mu$m and 40 $\mu$m, in particular between 3 $\mu$m and 15 $\mu$m;
wherein the fabric (102) is configured as a lotion delivery system;
wherein the fiber (108) network is tailored to control at least one function or property, in particular in terms of at least one of the group consisting of wicking, anisotropic behavior, oil retention, water retention, cleanability, and roughness.

9. The fabric (102) according to any of claims 1 to 8, wherein the fibers (108) have a copper content of less than 5 ppm and/or have a nickel content of less than 2 ppm.

10. A method of manufacturing nonwoven cellulose fiber fabric (102) directly from lyocell spinning solution (104), wherein the method comprises

extruding the lyocell spinning solution (104) through at least one jet (122) with orifices (126) supported by a gas flow (146) into a coagulation fluid (106) atmosphere to thereby form substantially endless fibers (108);
collecting the fibers (108) on a fiber support unit (132) to thereby form the fabric (102);
adjusting process parameters so that different ones of the fibers (108) are located at least partially in different distinguishable interconnected layers (200, 202), and so that the fabric (102) is optically transparent when wet.

11. The method according to claim 10, wherein the method further comprises further processing the fibers (108) and/or the fabric (102) *in situ* after collection on the fiber support unit (132), in particular by at least one of the group consisting of hydro-entanglement, needle punching, impregnation, steam treatment with a pressurized steam, and calendering.

12. A device (100) for manufacturing nonwoven cellulose fiber fabric (102) directly from lyocell spinning solution (104), wherein the device (100) comprises:

at least one jet (122) with orifices (126) configured for extruding the lyocell spinning solution (104) supported by a gas flow (146);
a coagulation unit (128) configured for providing a coagulation fluid (106) atmosphere for the extruded lyocell spinning solution (104) to thereby form substantially endless fibers (108);

a fiber support unit (132) configured for collecting the fibers (108) to thereby form the fabric (102);

a control unit (140) configured for adjusting process parameters so that different ones of the fibers (108) are located at least partially in different distinguishable interconnected layers (200, 202), and so that the fabric (102) is optically transparent when wet.

13. The device (100) according to claim 12, comprising a further jet (122) with orifices (126) configured for extruding further lyocell spinning solution (104) supported by a further gas flow (146), the further jet (12e) being arranged downstream of the jet (122), and wherein the jet (12e) is configured for forming one of the layers (202) and the further jet (122) is configured for forming another one of the layers (200) on top of the layer (202).

14. A method of using a nonwoven cellulose fiber fabric (102) according to any of claims 1 to 9 for at least one of the group consisting of a wipe, a dryer sheet, a filter, a hygiene product, a medical application product, a geotextile, an agrotextile, clothing, a product for building technology, an automotive product, a furnishing, an industrial product, a product related to beauty, leisure, sports or travel, and a product related to school or office.

15. A product or composite, comprising a fabric (102) according to any of claims 1 to 9.

Fig. 1

EP 3 385 430 A1

102

108

108

204

204

←300μm→

Fig. 2

102

108

108

204    204

Fig. 3

Fig. 4

Fig. 5

Fig. 6

Fig. 7

204 108

102

200

202

**Fig. 8**

204

D

204

108

204

108

100

122 ← 140 → 122

126

128 128 128 128

126 200 202 102

106

108 104 104 108 106

**Fig. 9**

108 108

102

200

200

202

108

**Fig. 10**

Fig. 11

Fig. 12

Fig. 13

Europäisches
Patentamt

European
Patent Office

Office européen
des brevets

# EUROPEAN SEARCH REPORT

Application Number

EP 17 16 4613

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 860 307 A1 (KURARAY KURAFLEX CO LTD [JP]) 15 April 2015 (2015-04-15) | 1,10,12 | INV. D04H3/013 B32B5/26 |
| Y | * paragraphs [0001], [0012], [0015], [0017], [0024], [0059], [0061] * | 2-9,11, 13 | |
| X | US 6 241 927 B1 (MUELLEDER EDUARD [AT] ET AL) 5 June 2001 (2001-06-05) | 10,12 | |
| Y | * column 3, lines 19-35 * <br> * column 4, lines 37-52 * | 2-9,11, 13 | |
| X,D | WO 98/07911 A1 (WEYERHAEUSER CO [US]) 26 February 1998 (1998-02-26) | 10,12 | |
| Y | * page 5, lines 1-36 * <br> * page 9, lines 6-37 * | 2-9,11, 13 | |
| X,D | WO 2007/124521 A1 (CHEMIEFASER LENZING AG [AT]; WHITE PAT [GB]; HARMS HAIO [AT]; HAYHURST) 8 November 2007 (2007-11-08) | 1,10,12, 14,15 | |
| Y | * page 2, line 1 - page 3, line 4 * <br> * page 7, line 5 - page 9 * | 2-9,11, 13 | |

TECHNICAL FIELDS
SEARCHED (IPC)

D04H
B32B

The present search report has been drawn up for all claims

| Place of search | Date of completion of the search | Examiner |
|---|---|---|
| Munich | 2 August 2017 | Lanniel, Geneviève |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 17 16 4613

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2017

| Patent document cited in search report | | Publication date | Patent family member(s) | | Publication date |
|---|---|---|---|---|---|
| EP 2860307 | A1 | 15-04-2015 | AU | 2013275328 A1 | 22-01-2015 |
| | | | CN | 104487629 A | 01-04-2015 |
| | | | EP | 2860307 A1 | 15-04-2015 |
| | | | JP | 6158178 B2 | 05-07-2017 |
| | | | JP | WO2013187404 A1 | 04-02-2016 |
| | | | KR | 20150030699 A | 20-03-2015 |
| | | | SG | 11201407928X A | 29-01-2015 |
| | | | TW | 201410269 A | 16-03-2014 |
| | | | US | 2015125499 A1 | 07-05-2015 |
| | | | WO | 2013187404 A1 | 19-12-2013 |
| US 6241927 | B1 | 05-06-2001 | AT | 239111 T | 15-05-2003 |
| | | | AT | 405531 B | 27-09-1999 |
| | | | AU | 740994 B2 | 22-11-2001 |
| | | | BR | 9806227 A | 21-03-2000 |
| | | | CA | 2263183 A1 | 23-12-1998 |
| | | | CN | 1236403 A | 24-11-1999 |
| | | | DE | 59808154 D1 | 05-06-2003 |
| | | | EP | 0918894 A1 | 02-06-1999 |
| | | | ES | 2198717 T3 | 01-02-2004 |
| | | | HK | 1023152 A1 | 02-04-2004 |
| | | | ID | 21230 A | 06-05-1999 |
| | | | JP | 3849062 B2 | 22-11-2006 |
| | | | JP | 2000517009 A | 19-12-2000 |
| | | | KR | 20000068155 A | 25-11-2000 |
| | | | MY | 125882 A | 30-08-2006 |
| | | | NO | 990731 A | 17-02-1999 |
| | | | PT | 918894 E | 29-08-2003 |
| | | | SI | 918894 T1 | 31-10-2003 |
| | | | TW | 544476 B | 01-08-2003 |
| | | | US | 6241927 B1 | 05-06-2001 |
| | | | WO | 9858103 A1 | 23-12-1998 |
| | | | ZA | 9805246 B | 08-01-1999 |
| WO 9807911 | A1 | 26-02-1998 | AT | 225418 T | 15-10-2002 |
| | | | BR | 9711352 A | 18-01-2000 |
| | | | CA | 2264180 A1 | 26-02-1998 |
| | | | CA | 2641970 A1 | 26-02-1998 |
| | | | CA | 2641972 A1 | 26-02-1998 |
| | | | CN | 1238015 A | 08-12-1999 |
| | | | CN | 1348023 A | 08-05-2002 |
| | | | CN | 1356412 A | 03-07-2002 |
| | | | CN | 1356413 A | 03-07-2002 |
| | | | CN | 1356414 A | 03-07-2002 |
| | | | DE | 69716092 D1 | 07-11-2002 |
| | | | DE | 69716092 T2 | 30-01-2003 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 17 16 4613

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2017

| Patent document cited in search report | Publication date | Patent family member(s) | | Publication date |
|---|---|---|---|---|
| | | EP | 0920548 A1 | 09-06-1999 |
| | | ES | 2185045 T3 | 16-04-2003 |
| | | HK | 1023377 A1 | 26-07-2002 |
| | | JP | 4018152 B2 | 05-12-2007 |
| | | JP | 4134209 B2 | 20-08-2008 |
| | | JP | 2001501260 A | 30-01-2001 |
| | | JP | 2007046223 A | 22-02-2007 |
| | | KR | 20000068304 A | 25-11-2000 |
| | | WO | 9807911 A1 | 26-02-1998 |
| WO 2007124521 A1 | 08-11-2007 | AT | 503625 A1 | 15-11-2007 |
| | | CN | 101432476 A | 13-05-2009 |
| | | CN | 103173938 A | 26-06-2013 |
| | | EP | 2013390 A1 | 14-01-2009 |
| | | EP | 2957667 A1 | 23-12-2015 |
| | | ES | 2553182 T3 | 04-12-2015 |
| | | JP | 5097771 B2 | 12-12-2012 |
| | | JP | 2009535521 A | 01-10-2009 |
| | | KR | 20080111521 A | 23-12-2008 |
| | | US | 2009186189 A1 | 23-07-2009 |
| | | WO | 2007124521 A1 | 08-11-2007 |

EPO FORM P0459

For more details about this annex : see Official Journal of the European Patent Office, No. 12/82

page 2 of 2

**REFERENCES CITED IN THE DESCRIPTION**

**Patent documents cited in the description**

- WO 9826122 A
- WO 9947733 A
- WO 9807911 A
- US 6197230 B
- WO 9964649 A
- WO 05106085 A
- EP 1358369 A
- EP 2013390 A
- WO 07124521 A1
- WO 07124522 A1